# EUROPÄISCHE PATENTSCHRIFT

(11) **EP 2 748 145 B1**
(45) Veröffentlichungstag und Bekanntmachung des Hinweises auf die Patenterteilung: **19.10.2016**
(21) Anmeldenummer: 12762544.0
(22) Anmeldetag: 22.08.2012
(51) Int. Cl.: C07D 207/04, H01G 11/06, H01G 11/58

(54) **SCHWEFELHALTIGE ADDITIVE FÜR ELEKTROCHEMISCHE ODER OPTOELEKTRONISCHE VORRICHTUNGEN**
SULFUR-CONTAINING ADDITIVES FOR ELECTROCHEMICAL OR OPTOELECTRONIC DEVICES
ADDITIFS CONTENANT DU SOUFRE POUR DISPOSITIFS ÉLECTROCHIMIQUES OU OPTOÉLECTRONIQUES

(30) Priorität: 24.08.2011 DE 102011111059
(43) Veröffentlichungstag der Anmeldung: 02.07.2014
(73) Patentinhaber: BASF SE, 67056 Ludwigshafen am Rhein (DE)
(72) Erfinder: SCHMIDT, Michael, 64342 Seeheim-Jugenheim (DE); IGNATYEV, Nikolai, 47058 Duisburg (DE); SEMRAU, Guenter, 63699 Kefenrod (DE); FRANK, Walter, 42329 Wuppertal (DE); BARTHEN, Peter, 47495 Rheinberg (DE); BREITENSTEIN, Christoph, 45476 Muelheim (DE)
(74) Vertreter: BASF IP Association
(86) Internationale Anmeldenummer: PCT/EP2012/066292
(87) Internationale Veröffentlichungsnummer: WO 2013/026854

(56) Entgegenhaltungen:
- WO-A2-2005/055286
- KING J F ET AL: "BETYLATES. 3. PREPARATIVE NUCLEOPHILIC SUBSTITUTION BY WAY OF [2]-, [3]-, AND [4]BETYLATES. STOICHIOMETRIC PHASE TRANSFER AND SUBSTRATE-REAGENT ION-PAIR (SRIP) REACTIONS OF BETYLATES", JOURNAL OF THE AMERICAN CHEMICAL SOCIETY, ACS PUBLICATIONS, US, Bd. 104, 1. Januar 1982 (1982-01-01), Seiten 7108-7122, XP002546399, ISSN: 0002-7863, DOI: 10.1021/JA00389A038

## Beschreibung

Die Erfindung betrifft schwefelhaltige Verbindungen der Formel I, deren Herstellung und deren Verwendung als Additive in elektrochemischen oder optoelektronischen Vorrichtungen, insbesondere in Elektrolyten für Lithium-Batterien, Lithium-Ionen-Batterien, Doppelschichtkondensatoren, Lithium-Ionen-Kondensatoren, Solarzellen, elektrochrome Displays, Sensoren und/oder Biosensoren.

Eine Schlüsselkomponente für elektrochemische oder optoelektronische Vorrichtungen ist der Elektrolyt. Dieser beeinflusst die Lebensdauer, Sicherheit und Leistung beispielsweise einer Lithium-Ionen-Zelle entscheidend.

In den letzten Jahren wurde insbesondere der Focus auf die Entwicklung von Additiven für elektrochemische Zellen gelegt, insbesondere für Lithium- und Lithium-Ionen-Batterien, sowie Doppelschichtkondensatoren. Praktisch jeder kommerziell eingesetzte Elektrolyt beinhaltet heute minimal ein Additiv.

Additive zielen dabei auf die Verbesserung chemischer und elektrochemischer Eigenschaften des Elektrolyten ab. Bereits kleine Mengen an Additiven können dabei die Eigenschaften des Elektrolyten signifikant verbessern. So beschäftigt sich eine Vielzahl von wissenschaftlichen Veröffentlichungen und Review-Artikeln mit Additiven (siehe z.B. Chem. Rev. 104, 4303-4417). Additive werden zum Beispiel eingesetzt, um die thermische Stabilität oder die Leitfähigkeit des Elektrolyten zu erhöhen, Verunreinigungen im Elektrolyten (z.B. Wasser oder HF) abzufangen, die Zyklenfestigkeit, Belastbarkeit und Lebensdauer einer elektrochemischen Zelle zu verbessern oder die Sicherheit der Batterie zu erhöhen.

Typischerweise werden hierfür meist organische Verbindungen wie Vinylencarbonat, Propansulton oder Vinylacetat (zur Verbesserung der Zyklenfestigkeit), Biphenyl (Überladeschutz), organische Amine (Abfangen von HF) oder verschiedene Sulphone (Verbesserung der thermischen Stabilität) eingesetzt.

Das Haupteinsatzgebiet für Additive ist die Optimierung des sogenannten "Solid Electrolyte Interface" (SEI), d.h. der Grenzfläche zwischen Elektrode und Elektrolyt. Diese SEI beeinflusst signifikant Zyklenfestigkeit, kalendarische Alterung und Belastbarkeit (Hochstromfestigkeit) der elektrochemischen oder elektrooptischen Vorrichtung.

Im Elektrolyt beispielsweise für Lithium-Ionen-Batterien, liegen die Lithium-Ionen nicht als "nackte" Kationen vor, sondern Sie sind von Lösemittel-Molekülen umhüllt. Diese sogenannte Solvat-Hülle vergrößert das kleine Lithium-Ion um ein Vielfaches.

Beim Laden einer Lithium-Ionen-Batterie enthaltend eine Graphit-Anode, dringen solvatisierte Lithium-Ionen in die äußeren Strukturen der Graphit-Anode ein. Da alle Lösungsmittel unter diesen extrem reduzierenden Bedingungen elektrochemisch instabil sind, zersetzen sie sich unter Bildung organischer Lithiumsalze. Die im Elektrolyten schwer löslichen Lithiumsalze lagern sich auf der Elektrode und in den äußeren Strukturen des Graphits ab und bilden dort jene Schicht, die als SEI bezeichnet wird (siehe z.B. Chem. Rev. 104, 4303-4417).

Diese für Lithium-Ionen durchlässige aber gleichzeitig elektronisch isolierende Schicht verhindert den direkten Kontakt zwischen Elektrode und Lösungsmittel. Eine weitere Zersetzung des Lösungsmittels wird somit verhindert. Zudem wirkt die SEI desolvatisierend, d.h. das Lithium-Ion streift beim Durchgang die Lösungsmittel-Moleküle ab und wandert als nacktes Kation in die Elektrode. Ohne diesen Effekt käme es zu einer signifikanten Aufweitung der Graphitschichten beim Laden und zu einer Kontraktion beim Entladen der Zelle. Dieses "Atmen" würde ohne eine geeignete SEI mit zunehmenden Lade/Entladezyklen zu einem "Zerbröseln" der Elektrode und somit zu einem schnellen Lebensende der Batterie führen.

Aufbau und Eigenschaften der SEI können signifikant durch Additive beeinflusst werden. Das Ziel ist es, eine Verbesserung der Zyklenfestigkeit zu erreichen, ohne eine Verschlechterung des Innenwiderstandes zu erzeugen.

Aufgabe der vorliegenden Erfindung war es daher, neue Additive für Elektrolyte zu entwickeln, die schneller und gezielter mit geladenen Oberflächen reagieren und damit beispielsweise für elektrochemische Vorrichtungen die Bildung der SEI positiv zu beeinflussen.

Die Aufgabe wird erfindungsgemäß durch die schwefelhaltigen Verbindungen der Formel I, wie nachfolgend beschrieben, gelöst.

Ein erster Gegenstand der Erfindung sind daher Verbindungen der Formel (I)

[K-(CH₂)-(CH₂)ᵤ-SO₃-(CH₂)ᵥ-R"]⁺ [A]- (I),

wobei
u 0, 1, 2, 3, 4, 5, 6 oder 7 bedeutet und wobei mindestens eine CH₂-Gruppe der -(CH₂)ᵤ-Alkylenkette mit u = 2, 3, 4, 5, 6 oder 7 durch O ersetzt sein kann oder mindestens eine Doppelbindung enthalten kann,
v 0, 1, 2, 3 oder 4 bedeutet, 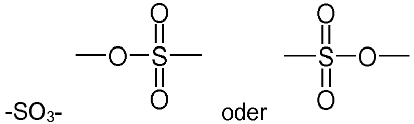 bedeutet,
   R" jeweils unabhängig voneinander eine geradkettige oder verzweigte Alkylgruppe mit 1 bis 8 C-Atomen, die nicht fluoriert, teilweise fluoriert oder vollständig fluoriert sein kann, eine geradkettige oder verzweigte Alkenylgruppe mit 2 bis 20 C-Atomen und einer oder mehrerer Doppelbindungen, eine geradkettige oder verzweigte Alkinylgruppe mit 2 bis 20 C-Atomen und einer oder mehrerer Dreifachbindungen oder eine Arylgruppe mit 6 bis 12 C-Atomen, die durch F, Cl und/oder eine geradkettige oder verzweigte, teilweise fluorierte oder vollständig fluorierte Alkylgruppe mit 1 bis 8 C-Atomen einfach oder mehrfach subsituiert sein kann, bedeutet,
   K ein Kation ist, ausgewählt aus der Gruppe R₃N⁺-*, R₃P⁺-*, 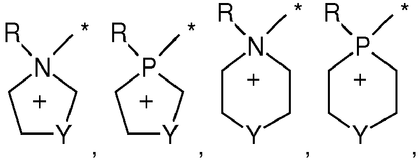 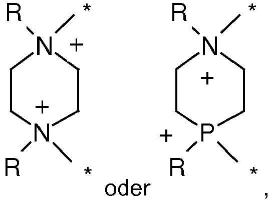 wobei
   R jeweils unabhängig voneinander Methyl, Ethyl, n-Propyl, iso-Propyl, n-Butyl, sec.-Butyl oder tert.-Butyl bedeutet, wobei mindestens eine, nicht direkt mit N oder P verbundene, CH₂-Gruppe der genannten Reste R durch O ersetzt sein kann,
   - * die Bindung von K zur (CH₂)-Gruppe in Formel (II) bedeutet,
   Y CH₂, O, S oder NR' bedeutet und
   R' Methyl, Ethyl, n-Propyl, iso-Propyl, n-Butyl, sec.-Butyl oder tert.-Butyl bedeutet,
   A ein Anion ist, ausgewählt aus der Gruppe
   [F_{z}B(CₘF_{2m,1})_{4-z}]⁻,
   [F_{y}P(CₘF₂ₘ₊₁)_{6-y}]⁻,
   [(CₘF₂ₘ₊₁)₂P(O)O]⁻,
   [CₘF₂ₘ₊₁P(O)O₂]²⁻,
   [O-C(O)-CₘF₂ₘ₊₁]⁻,
   [N(C(O)-CₘF₂ₘ₊₁)₂]⁻,
   [N(S(O)₂-CₘF₂ₘ₊₁)₂]⁻,
   [N(C(O)-CₘF₂ₘ₊₁)(S(O)₂-CₘF₂ₘ₊₁)]⁻,
   [N(C(O)-CₘF₂ₘ₊₁)(C(O)F)]⁻,
   [N(S(O)₂-CₘF₂ₘ₊₁)(S(O)₂F)]⁻,
   [N(S(O)₂F)₂]⁻,
   [C(C(O)-CₘF₂ₘ₊₁)₃]⁻,
   [C(S(O)₂-CₘF₂ₘ₊₁)₃]⁻, 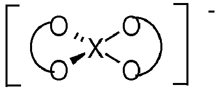 und 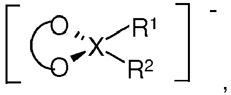 wobei
      m 1, 2, 3, 4, 5, 6, 7 oder 8 bedeutet, wobei teilweise CF₂-Gruppen in den genannten Anionen durch O, S(O)₂, NR oder CH₂ ersetzt sein können,
      z 1, 2, oder 3 bedeutet, y 1, 2, 3, 4, 5 oder 6 bedeutet,
      X B oder Al bedeutet,
      R¹ und R² jeweils unabhängig voneinander F, Cl, Br, I, eine geradkettige oder verzweigte Perfluoralkylgruppe mit 1 bis 20 C-Atomen, eine geradkettige oder verzweigte Alkoxygruppe mit 1 bis 20 C-Atomen, die nicht fluoriert, teilweise fluoriert oder vollständig fluoriert sein kann, oder - O-C(O)-Alkyl bedeutet, wobei Alkyl eine geradkettige oder verzweigte Alkylgruppe mit 1 bis 20 C-Atomen bedeutet, die nicht fluoriert, teilweise fluoriert oder perfluoriert sein kann,
         und wobei 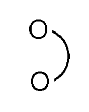 jeweils unabhängig voneinander ein zweizähniger Rest bedeutet, der sich von einem 1,2- oder 1,3- Diol, von einer 1,2 -oder 1,3- Dicarbonsäure oder einer 1,2- oder 1,3- Hydroxycarbonsäure ableitet, in dem jeweils zwei benachbarte OH-Gruppen der entsprechenden Verbindung mit dem Zentralatom X jeweils eine Bindung eingehen und dabei zwei H-Atome formal abgespaltet werden und wobei die Elektroneutralität des entsprechenden Salzes der Formel (I) zu beachten ist. Für den Fall, dass das Kation K 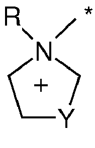 ist, kann [A]- auch ausgewählt sein aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4.

Die erfindungsgemäßen Verbindungen der Formel (I), wie zuvor beschrieben, bilden bei Potentialen zwischen ca. 2,5 V und 0,9 V gegen Li/Li⁺ eine passivierende Deckschicht auf der negativen Elektrode, die sogenannte SEI, wie zuvor beschrieben.

Eine überraschende und herausragende Eigenschaft dieser Verbindungen der Formel (I) ist, dass sie als Additive in Elektrolyten eingesetzt, im allgemeinen nicht zu einer Erhöhung des Innenwiderstandes der elektrochemischen Zelle führen wie z.B. Vinylencarbonat oder Propansulton.

Da es sich bei den Verbindungen der Formel (I) um Salze handelt, sind sie in der Regel nicht flüchtig und besitzen keinen messbaren Dampfdruck. Dies ist ein weiterer Vorteil gegenüber den bisher eingesetzten organischen Additiven, beispielsweise Propansulton.

Ähnliche Verbindungen zu den Verbindungen der Formel (I) sind in H. Distler und E.-H. Pommer, Erdöl und Kohle, 1965, 18(5), 381-386 beschrieben. Die quartären Ammoniumsalze der beschriebenen Taurinester sind wasserlösliche mikrozide Verbindungen, insbesondere zur Algenbekämpfung. Das Gegenion dieser quartären Ammoniumsalze ist Methylsulfat, beispielsweise in der Verbindung TAS, auch unter dem Markennamen Sepacid® bekannt, d.h. das Tetramethylammoniumsalz des Taurinphenylesters oder auch synonym dazu Phenyloxysulfonylethyl-trimethylammoniummethylsulfat.

In DE 4211140 werden ähnliche Phosphoniumsalze und ihre Verwendung als Glanzmittel für wässrig-saure galvanische Nickelbäder beschrieben, beispielsweise Triphenylphosphoniumessigsäurebenzylesterchlorid, 3-(Triphenylphosphonium)propyl-methylketonchlorid, 4-(Triphenyl-phosphonium)buttersäure-chlorid, 4-(Triphenylphosphonium)acetessigsäuremethylester-chlorid, 5-(Triphenylphosphonium)valeriansäureethylesterbromid oder Triphenylphosphoniumallylchlorid. Quartäre Phosphoniumsalze von Sulfonsäureestern mit dem Gegenion Chlorid oder Bromid werden nicht beschrieben.

Xun He und Tak Hang Chan, Tetrahedron, 2006, 62, 3389-3394 beschreiben nicht-flüchtige und geruchsarme Organoschwefelverbindungen, die an Ionischen Flüssigkeiten verankert sind, als recyclisierbare Reagenzien für die Swern-Oxidation, beispielsweise die Verbindungen

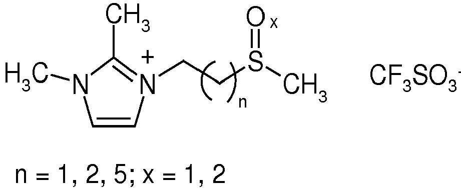

In WO 2005/055286 werden zwitterionische Verbindungen der Formel 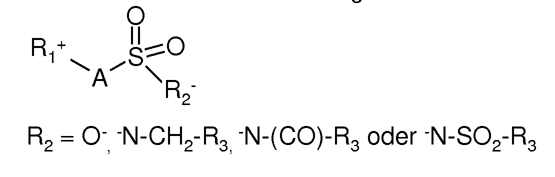 beschrieben, wobei R₁⁺ ein heterocyclisches Kation, ein Ammoniumkation, ein Guanidiniumkation oder ein Phosphoniumkation bedeutet, A ein Spacer und R₃ H, Alkyl, Cycloalkyl, Heterocyclyl, Alkenyl, Alkinyl oder Aryl bedeutet, zur Verwendung in einer Solarzelle als Bestandteil von Ladungstransportschichten. Repräsentativ werden die folgenden Verbindungen beschrieben: 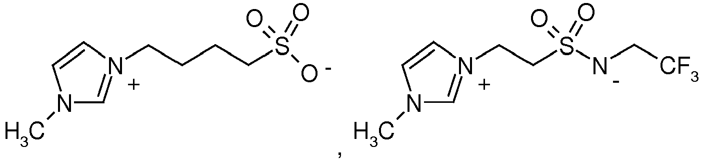 oder

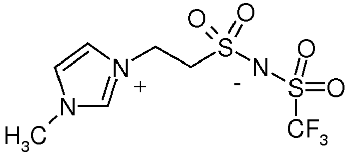

WO 2006/017898 beschreibt zwitterionische Additive für elektrochemische Vorrichtungen der allgemeinen Struktur Kation-Spacer-Anion. Repräsentative Verbindungen für diese Zwitterionen, d.h. für Verbindungen, die sowohl eine positive Ladung als auch eine negative Ladung in einem Molekül tragen, sind die Verbindungen N-Methyl-N-(n-butansulfonat)pyrrolidinium 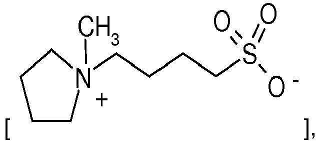 N-Methyl-N-(n-propanesulfonat)pyrrolidinium oder 1-Butylimidazolium-3-(n-butansulfonat)

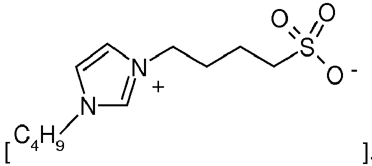

Eine geradkettige oder verzweigte Alkylgruppe mit 1 bis 20 C-Atomen ist beispielsweise Methyl, Ethyl, Isopropyl, Propyl, Butyl, sec.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl oder Eicosyl, welche gegebenenfalls auch teilweise fluoriert oder vollständig fluoriert (synonym dazu perfluoriert) sein kann. Der Ausdruck "perfluoriert" gibt an, dass alle H-Atome in der angegebenen Alkylgruppe durch F-Atome substituiert sind. Der Ausdruck " teilweise fluoriert" gibt an, dass mindestens ein H-Atom in der angegebenen Alkylgruppe durch ein F-Atom substituiert ist. Beispiele für teilweise fluorierte oder perfluorierte Alkylgruppen sind Difluormethyl, Trifluormethyl, Pentafluorethyl, Heptafluorpropyl oder Nonafluorbutyl.

Eine geradkettige oder verzweigte Alkenylgruppe mit 2 bis 20 C-Atomen, in dem auch mehrere Doppelbindungen vorhanden sein können, ist beispielsweise Allyl, 2- oder 3-Butenyl, iso-Butenyl, sec.-Butenyl, ferner 4-Pentenyl, iso-Pentenyl, Hexenyl, Heptenyl, Octenyl, -C₉H₁₇,-C₁₀H₁₉ bis -C₂₀H₃₉; vorzugsweise Allyl, 2- oder 3-Butenyl, iso-Butenyl, sec.-Butenyl, ferner bevorzugt 4-Pentenyl, iso-Pentenyl oder Hexenyl.

Eine geradkettige oder verzweigte Alkinylgruppe mit 2 bis 20 C-Atomen, in dem auch mehrere Dreifachbindungen vorhanden sein können, ist beispielsweise Ethinyl, 1- oder 2-Propinyl, 2-oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Eine geradkettige oder verzweigte Alkoxygruppe mit 1 bis 20 C-Atomen ist beispielsweise Methoxy, Ethoxy, iso-Propoxy, Propoxy, Butoxy, sec.-Butoxy oder tert.-Butoxy, ferner auch Pentoxy, 1-, 2- oder 3-Methylbutoxy, 1,1-, 1,2- oder 2,2-Dimethylpropoxy, 1-Ethylpropoxy, Hexyloxy, Heptyloxy, Octyloxy, Nonyloxy, Decyloxy, Undecyloxy, Dodecyloxy, Tridecyloxy, Tetradecyloxy, Pentadecyloxy, Hexadecyloxy, Heptadecyloxy, Octadecyloxy, Nonadecyloxy oder Eicosyloxy, die nicht fluoriert, teilweise fluoriert oder vollständig fluoriert sein kann. Der Ausdruck " teilweise fluoriert" gibt an, dass mindestens ein H-Atom in der angegebenen Alkoxygruppe durch ein F-Atom substituiert ist. Der Ausdruck "vollstänldig fluoriert" gibt an, dass alle H-Atome in der angegebenen Alkoxygruppe durch F-Atome substituiert sind.

Eine Arylgruppe mit 6 bis 12 C-Atomen ist beispielsweise Phenyl, Naphthyl oder Phenanthryl, die durch F, Cl und/oder einer geradkettigen oder verzweigten, teilweise fluorierten oder vollständig fluorierten Alkylgruppe mit 1 bis 8 C-Atomen einfach oder mehrfach subsituiert sein kann, bevorzugt Phenyl, welches durch F, Cl und/oder eine geradkettigen oder verzweigte, teilweise fluorierte oder vollständig fluorierte Alkylgruppe mit 1 bis 8 C-Atomen einfach oder mehrfach subsituiert sein kann, beispielsweise o-, m- oder p-Methylphenyl, o-, m- oder p-Ethylphenyl, o-, m- oder p-Propylphenyl, o-, m- oder p-(iso-Propyl)phenyl, o-, m- oder p-(tert.-Butyl)phenyl, o-, m-, p-(Trifluormethyl)phenyl, o-, m-, p-(Pentafluorethyl)phenyl, o-, m-, p-(Nonafluorbutyl)phenyl, o-, m- oder p-Fluorphenyl, o-, m- oder p-Chlorphenyl, 2,3-, 2,4-, 2,5-, 2,6-, 3,4- oder 3,5-Bis(trifluormethyl)phenyl, 2,3-, 2,4-, 2,5-, 2,6-, 3,4- oder 3,5-Bis(pentafluorethyl)phenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 5-Fluor-2-trifluormethylphenyl, 3,4,5-Trifluorphenyl oder 2,4,5-Trifluorphenyl. Besonders bevorzugt bedeutet die Arylgruppe mit 6 bis 12 C-Atomen Phenyl, welches durch F und/oder eine geradkettige oder verzweigte teilweise fluorierte oder perfluorierte Alkylgruppe mit 1 bis 8 C-Atomen einfach oder mehrfach substituiert sein kann.

Die Darstellung 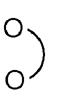 bedeutet jeweils unabhängig voneinander einen zweizähnigen Rest, der sich von einem 1,2- oder 1,3- Diol, von einer 1,2 -oder 1,3- Dicarbonsäure oder einer 1,2- oder 1,3- Hydroxycarbonsäure ableitet, in dem jeweils zwei benachbarte OH-Gruppen der entsprechenden Verbindung mit dem Zentralatom X jeweils eine Bindung eingehen und dabei zwei H-Atome formal abgespaltet werden.

Geeignete 1,2- oder 1,3-Diole zur Erzeugung des zweizähnigen Restes, wie zuvor beschrieben, sind sowohl aliphatisch als auch aromatisch, beispielsweise 1,2-Dihydroxybenzol (Catechol, Brenzkatechin), Propan-1,2-diol, Butan-1,2-diol, Propan-1,3-diol, Butan-1,3-Diol, Cyclohexyltrans-1,2-diol oder Naphthalen-2,3-diol, die gegebenenfalls mit F und/oder mindestens einer geradkettigen oder verzweigten, nicht fluorierten, teilweise fluorierten oder perfluorierten Alkylgruppe mit 1 bis 4 C-Atomen einfach oder mehrfach substituiert sein können. Ein Beispiel für derartig substituierte 1,2- oder 1,3-Diole ist 1,1,2,2-Tetra(trifluormethyl)-1,2-ethandiol.

Geeignete 1,2- oder 1,3-Dicarbonsäuren zur Erzeugung des zweizähnigen Restes, wie zuvor beschrieben, sind sowohl aliphatisch als auch aromatisch, beispielsweise Oxalsäure, Malonsäure (Propan-1,3-dicarbonsäure), Phthalsäure oder Isophthalsäure, die gegebenenfalls mit F und/oder mindestens einer geradkettigen oder verzweigten, nicht fluorierten, teilweise fluorierten oder perfluorierten Alkylgruppe mit 1 bis 4 C-Atomen einfach oder mehrfach substituiert sein können.

Geeignete 1,2- oder 1,3-Hydroxycarbonsäuren zur Erzeugung des zweizähnigen Restes, wie zuvor beschrieben, sind sowohl aliphatisch als auch aromatisch, beispielsweise Salicylsäure, Tetrahydrosalicylsäure, Äpfelsäure, 2-Hydroxyessigsäure, die gegebenenfalls mit F und/oder mindestens einer geradkettigen oder verzweigten, nicht fluorierten, teilweise fluorierten oder perfluorierten Alkylgruppe mit 1 bis 4 C-Atomen einfach oder mehrfach substituiert sein können. Ein Beispiel für derartig substituierte 1,2- oder 1,3-Hydroxycarbonsäuren ist 2,2-Bis(trifluormethyl)-2-hydroxy-essigsäure.

Die Variable u bedeutet 0, 1, 2, 3, 4, 5, 6 oder 7, bevorzugt steht u für 1, 2 oder 3.

Mindestens eine CH₂-Gruppe der-(CH₂)ᵤ-Alkylenkette mit u = 2, 3, 4, 5, 6 oder 7 kann durch O ersetzt sein oder kann mindestens eine Doppelbindung enthalten. Bevorzugt ist die -(CH₂)ᵤ-Alkylenkette unsubstituiert und u hat eine der zuvor angegebenen oder bevorzugt angegebenen Bedeutungen.

Die Variable v bedeutet 0, 1, 2, 3 oder 4, bevorzugt steht v für 0. Bevorzugt steht -SO₃- für

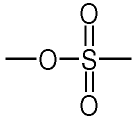

In den Verbindungen der Formel I steht R" bevorzugt jeweils unabhängig voneinander für eine geradkettige oder verzweigte Alkylgruppe mit 1 bis 8 C-Atomen, die nicht fluoriert, teilweise fluoriert oder vollständig fluoriert sein kann, eine geradkettige oder verzweigte Alkenylgruppe mit 2 bis 4 C-Atomen und einer oder mehrerer Doppelbindungen oder eine geradkettige oder verzweigte Alkinylgruppe mit 2 bis 4 C-Atomen und einer oder mehrerer Dreifachbindungen, beispielsweise Methyl, Ethyl, Trifluormethyl, Pentafluorethyl, n-Propyl, n-Butyl, n-Hexyl, n-Octyl, Ethenyl, Ethinyl, Allyl oder Prop-1-in-yl.

Besonders bevorzugt steht R" jeweils unabhängig voneinander für eine geradkettige oder verzweigte Alkylgruppe mit 1 bis 8 C-Atomen, die nicht fluoriert, teilweise fluoriert oder vollständig fluoriert sein kann. Ganz besonders bevorzugt steht R" für Methyl.

Die Variable Y in den Formeln für die Kationen K, wie zuvor beschrieben, bedeutet CH₂, O, S oder NR', wobei R' Methyl, Ethyl, n-Propyl, iso-Propyl, n-Butyl, sec.-Butyl oder tert.-Butyl bedeutet, besonders bevorzugt bedeutet Y CH₂.

Der Substituent R in den Formeln für die Kationen K, wie zuvor beschrieben, bedeutet bevorzugt Methyl, Ethyl, n-Propyl oder n-Butyl, besonders bevorzugt Methyl.

In den Verbindungen der Formel I steht K bevorzugt für das Kation 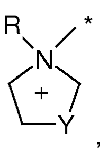 wobei Y und R eine der zuvor angegebenen Bedeutungen haben, ganz besonders bevorzugt für das Pyrrolidiniumkation 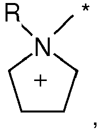 wobei R eine der zuvor genannten Bedeutungen hat.

Die Variable m in den Formeln der Anionen A, wie zuvor beschrieben, bedeutet bevorzugt 1, 2, 3, 4, 5, 6, 7 oder 8, besonders bevorzugt 2, 3 oder 4, ganz besonders bevorzugt 2 oder 4.

Die Variable y in den Formeln der Anionen A, wie zuvor beschrieben, bedeutet 1, 2, 3, 4, 5 oder 6, bevorzugt 3, 4 oder 5, besonders bevorzugt 3 oder 5, ganz besonders bevorzugt 3.

Die Variable z in den Formeln der Anionen A, wie zuvor beschrieben, bedeutet 1, 2, 3 oder 4, bevorzugt 1, 2 oder 3, ganz besonders bevorzugt 3.

Die Variable X in den Formeln der Anionen A, wie zuvor beschrieben, bedeutet B oder Al, bevorzugt B.

In den Verbindungen der Formel I steht A bevorzugt für ein Anion, ausgewählt aus der Gruppe
[F_{z}B(CₘF₂ₘ₊₁)_{4-z}]⁻,
[F_{y}P(CₘF₂ₘ₊₁)_{6-y}]⁻,
[O-C(O)-CₘF₂ₘ₊₁]⁻,
[N(S(O)₂-CₘF₂ₘ₊₁)₂]⁻,
[N(S(O)₂F)₂]⁻, 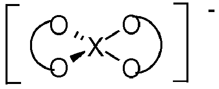 oder 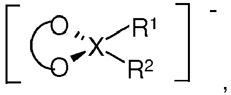 wobei
m 1, 2, 3, 4, 5, 6, 7 oder 8 bedeutet,
z 1, 2 oder 3 bedeutet,
y 3, 4, 5 oder 6 bedeutet,
X B bedeutet,
R¹ und R² jeweils unabhängig voneinander F, eine geradkettige oder verzweigte Perfluoralkylgruppe mit 1 bis 4 C-Atomen, eine geradkettige oder verzweigte Alkoxygruppe mit 1 bis 4 C-Atomen oder -O-C(O)-Alkyl bedeutet, wobei Alkyl eine geradkettige oder verzweigte Alkylgruppe mit 1 bis 20 C-Atomen bedeutet, die nicht fluoriert, teilweise fluoriert oder perfluoriert sein kann,
und wobei 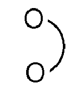 jeweils unabhängig voneinander ein zweizähniger Rest bedeutet, der sich von einem 1,2- oder 1,3- Diol, von einer 1,2 -oder 1,3- Dicarbonsäure oder einer 1,2- oder 1,3- Hydroxycarbonsäure ableitet, in dem jeweils zwei benachbarte OH-Gruppen der entsprechenden Verbindung mit dem Zentralatom X jeweils eine Bindung eingehen und dabei zwei H-Atome formal abgespaltet werden.

In den Verbindungen der Formel I steht A besonders bevorzugt für ein Anion, ausgewählt aus der Gruppe [F_{y}P(CₘF₂ₘ₊₁)₆₋y]⁻, 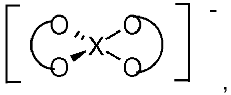 wobei
- m: 2,3 oder 4 bedeutet,
- y: 3, 4 oder 5 bedeutet,
- X: B bedeutet,
und wobei 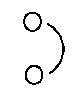 jeweils unabhängig voneinander ein zweizähniger Rest bedeutet, der sich von einem 1,2- oder 1,3- Diol, von einer 1,2 -oder 1,3- Dicarbonsäure oder einer 1,2- oder 1,3- Hydroxycarbonsäure ableitet, in dem jeweils zwei benachbarte OH-Gruppen der entsprechenden Verbindung mit dem Zentralatom X jeweils eine Bindung eingehen und dabei zwei H-Atome formal abgespaltet werden.

Bevorzugte Anionen der Formel [F_{z}B(CₘF₂ₘ₊₁)_{4-z}]⁻ sind die Anionen [F₃B(CF₃)]⁻ oder [F₃B(C₂F₅)]⁻.

Bevorzugte Anionen der Formel [F_{y}P(CₘF₂ₘ₊₁)₆-y]⁻ sind die Anionen [PF₆]⁻, [F₃P(C₂F₅)₃]⁻, [F₃P(C₃F₇)₃]⁻, [F₃P(C₄F₉)₃]⁻, [F₄P(C₂F₅)₂]⁻, [F₄P(C₃F₇)₂]⁻, [F₄P(C₄F₉)₂]⁻, [F₅P(C₂F₅)]⁻, [F₅P(C₃F₇)]⁻ oder [F₅P(C₄F₉)]⁻, besonders bevorzugt [PF₆]⁻, [F₃P(C₂F₅)₃]⁻, [F₃P(C₃F₇)₃]⁻, [F₃P(C₄F₉)₃]⁻, [F₅P(C₂F₅)]⁻, [F₅P(C₃F₇)]⁻ oder [F₅P(C₄F₉)]⁻, ganz besonders bevorzugt [F₃P(C₂F₅)₃]⁻ und [F₅P(C₂F₅)]⁻.

Bevorzugte Anionen der Formel [(CₘF₂ₘ₊₁)₂P(O)O]⁻ sind die Anionen [(C₂F₅)₂P(O)O]⁻, [(C₃F₇)₂P(O)O]⁻ oder [(C₄F₉)₂P(O)O]⁻.

Bevorzugte Anionen der Formel [CₘF₂ₘ₊₁P(O)O₂]²⁻ sind die Anionen [C₂F₅P(O)O₂]²⁻, [C₃F₇P(O)O₂]²⁻ oder [C₄F₉P(O)O₂]²⁻.
Bevorzugte Anionen der Formel [O-C(O)CₘF₂ₘ₊₁]⁻ sind die Anionen [O-C(O)CF₃]⁻, [O-C(O)C₂F₅]⁻ oder [O-C(O)C₄F₉]⁻, besonders bevorzugt [O-C(O)CF₃]⁻.

Bevorzugte Anionen der Formel [O-S(O)₂CₘF₂ₘ₊₁]⁻ sind die Anionen [O-S(O)₂CF₃]⁻ oder [O-S(O)₂C₂F₅]⁻, besonders bevorzugt [O-S(O)₂CF₃]⁻.

Bevorzugte Anionen der Formel [N(C(O)CₘF₂ₘ₊₁)₂]⁻ sind die Anionen [N(C(O)C₂F₅)₂]⁻ oder [N(C(O)(CF₃)₂]⁻.

Bevorzugte Anionen der Formel [N(S(O)₂CₘF₂ₘ₊₁)₂]⁻ sind die Anionen [N(S(O)₂CF₃)₂]⁻, [N(S(O)₂C₂F₅)₂]⁻, [N(S(O)₂C₃F₇)₂]⁻, [N(S(O)₂CF₃) (S(O)₂C₂F₅)]⁻ oder [N(S(O)₂C₄F₉)₂]⁻, besonders bevorzugt [N(S(O)₂CF₃)₂]⁻.

Bevorzugte Anionen der Formel [N(C(O)CₘF₂ₘ₊₁)(S(O)₂CₘF₂ₘ₊₁)]⁻ sind die Anionen [N(C(O)CF₃)(S(O)₂CF₃)]⁻, [N(C(O)C₂F₅)(S(O)₂CF₃)]⁻ oder [N(C(O)CF₃)(S(O)₂-C₄F₉)]⁻.

Bevorzugte Anionen der Formel [N(C(O)CₘF₂ₘ₊₁)(C(O)F)]⁻ sind die Anionen [N(C(O)CF₃)(C(O)F)]⁻, [N(C(O)C₂F₅)(C(O)F)]⁻ oder [N(C(O)C₃F₇)(C(O)F)]⁻.

Bevorzugte Anionen der Formel [N(S(O)₂CₘF₂ₘ₊₁)(S(O)₂F)]⁻ sind die Anionen [N(S(O)₂CF₃)(S(O)₂F)]⁻, [N(S(O)₂C₂F₅)(S(O)₂F)]⁻ oder [N(S(O)₂C₄F₉)(S(O)₂F)]⁻.

Bevorzugte Anionen der Formel [C(C(O)CₘF₂ₘ₊₁)₃]⁻ sind die Anionen [C(C(O)CF₃)₃]⁻, [C(C(O)C₂F₅)₃]⁻ oder [C(C(O)C₃F₇)₃]⁻.

Bevorzugte Anionen der Formel [C(S(O)₂CₘF₂ₘ₊₁)₃]⁻ sind die Anionen [C(S(O)₂CF₃)₃]⁻, [C(S(O)₂C₂F₅)₃]⁻ oder [C(S(O)₂C₄F₉)₃]⁻.

Bevorzugte Anionen der Formel 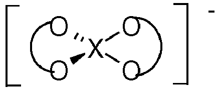 sind die Anionen Bis(catecholato)borat, wobei beide zweizähnige Reste auf 1,2-Dihydroxybenzol basieren, Bis(oxalato)borat, wobei beide zweizähnigen Reste auf Oxalsäure basieren, Bis(malonato)borat, wobei beide zweizähnigen Reste auf Malonsäure basieren, Malonatooxalatoborat, wobei ein zweizähniger Rest auf Malonsäure und ein zweizähniger Rest auf Oxalsäure basiert, Naphtholatooxalatoborat, wobei ein zweizähniger Rest auf Naphthalen-2,3-diol und ein zweizähniger Rest auf Oxalsäure basiert, Bis(naphtholato)borat, wobei beide zweizähnigen Reste auf Naphthalen-2,3-diol basieren oder Bis(salicylato)borat, wobei beide zweizähnigen Reste auf Salicylsäure basieren, ganz besonders bevorzugt Bis(oxalato)borat, abgekürzt BOB.

Bevorzugte Anionen der Formel 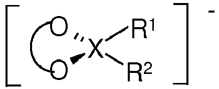 sind die Anionen Difluor-oxalato-borat, Di-Trifluoracetato-oxalato-borat, Difluor-di-(trifluormethyl)glykonato-borat, wobei der zweizähnige Rest auf 2,2-Bis(trifluormethyl)-2-hydroxy-essigsäure basiert, Difluor-perfluorpinakolatoborat, wobei der zweizähnige Rest auf 1,1,2,2-Tetra(triflukormethyl)-1,2-ehtandiol basiert, Trifluormethyl-methoxy-oxalato-borat, Pentafluorethyl-methoxy-oxalato-borat, Trifluormethyl-methoxy-catecholato-borat, Pentafluorethylmethoxy-catecholato-borat, Trifluormethyl-methoxy-malonato-borat, Pentafluorethyl-methoxy-malonato-borat, Pentafluorethyl-methoxy-naphtholato-borat oder Fluor-pentafluorethyl-oxalato-borat, besonders bevorzugt Difluor-oxalato-borat.

Besonders bevorzugte Verbindungen der Formel I sind die Verbindungen

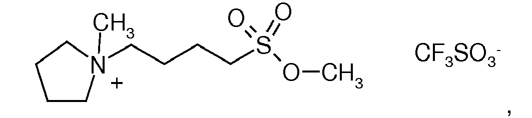

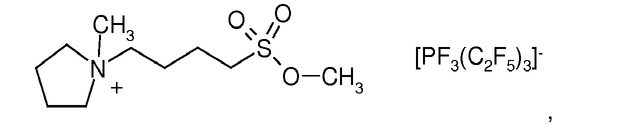

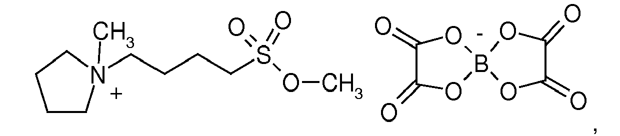

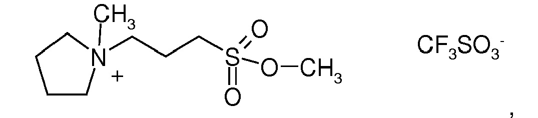

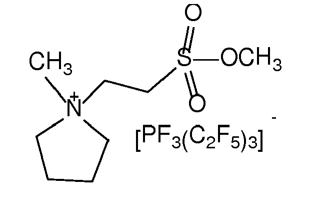

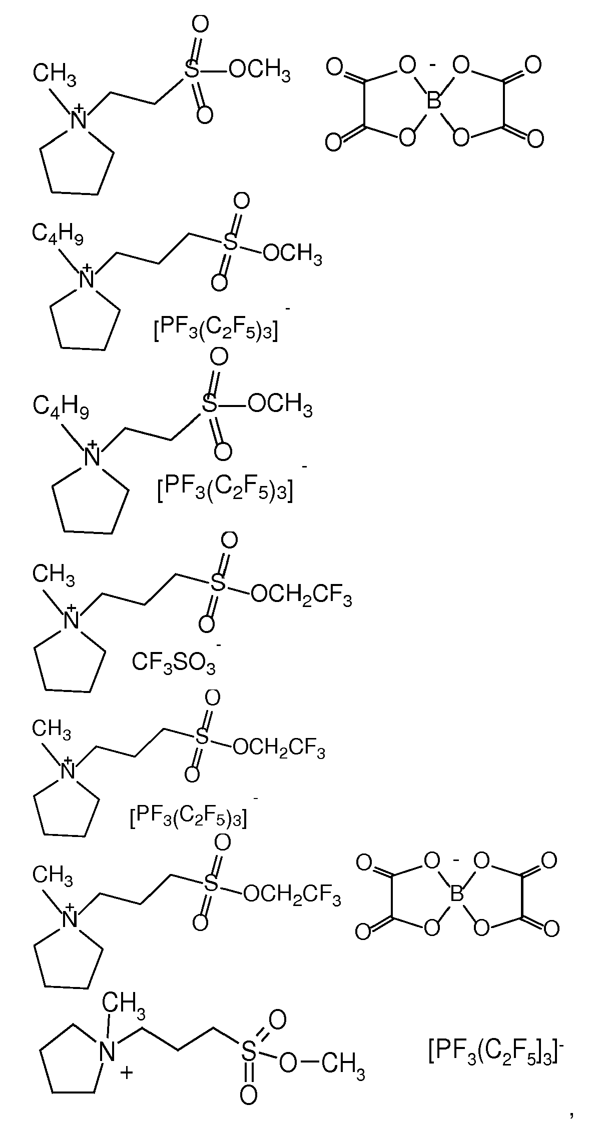

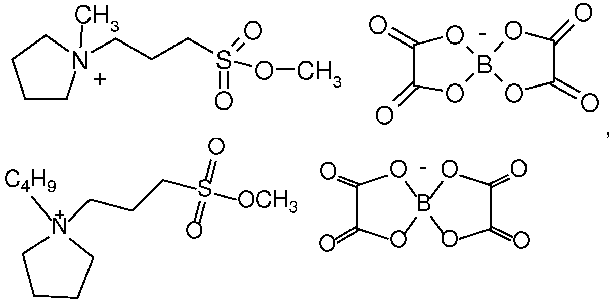

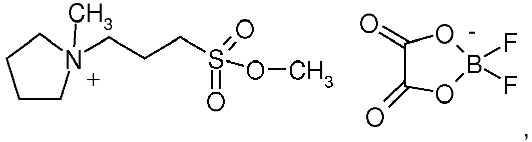

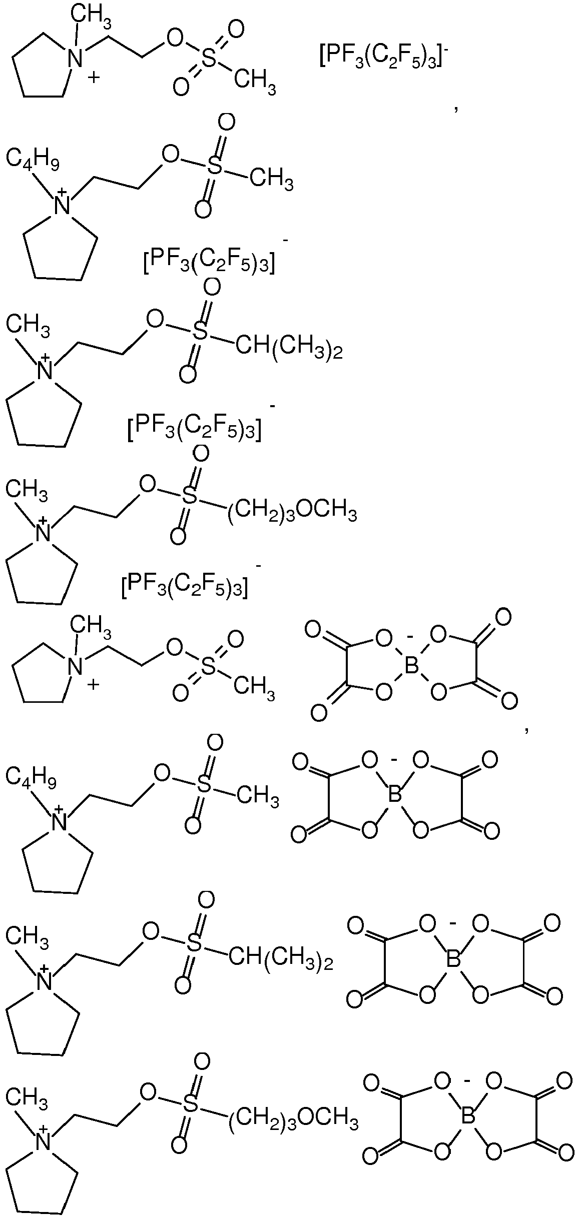

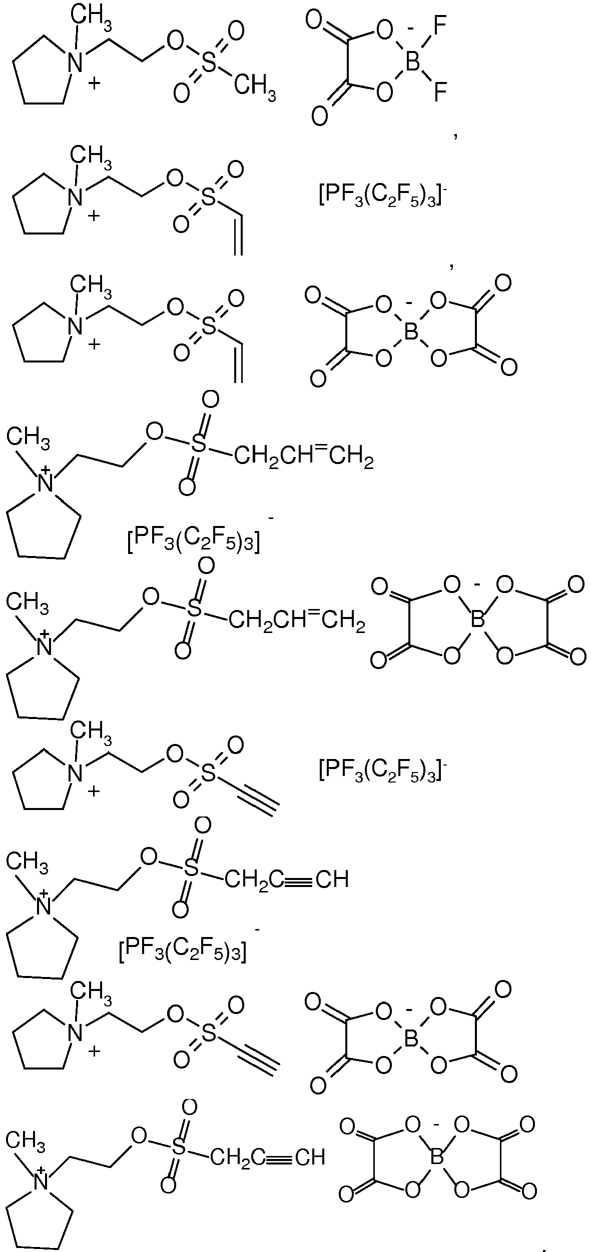

Es versteht sich für den Fachmann von selbst, dass in den erfindungsgemäßen Verbindungen Substituenten wie beispielsweise C, H, N, O, Cl, F durch die entsprechenden Isotope ersetzt sein können.

Die Verbindungen der Formel I, wie zuvor beschrieben werden in der Regel ausgehend von den entsprechenden korrespondierenden Aminen oder Phosphinen synthetisiert.

Die mehrstufigen Synthesen, die nachfolgend beschrieben werden, können in Einzelschritten unter Isolierung und Aufreinigung der entsprechend angegebenen Zwischenverbindungen erfolgen oder nachfolgend, indem lediglich die weiteren Reaktionsbedingungen entsprechend angepasst werden.
Die Verfahrensschritte können an der Luft, vorzugsweise in trockener Atmosphäre, zum Beispiel unter trockener Luft, Stickstoff oder Argon, durchgeführt werden.
Ein weiterer Gegenstand der Erfindung ist daher ein Verfahren zur Herstellung von Verbindungen der Formel I, wie zuvor beschrieben oder als bevorzugt beschrieben, wobei SO₃ der Teilstruktur 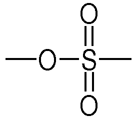 entspricht, dadurch gekennzeichnet, dass eine Verbindung der Formel (II)

K-1 (II),

wobei K-1 ausgewählt wird aus der Gruppe R₃N, R₃P, 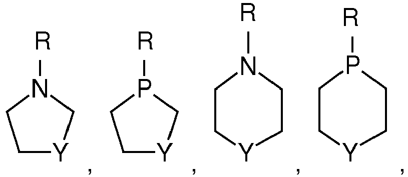 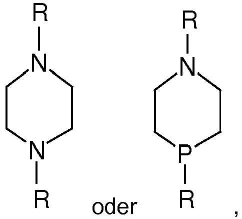 wobei
R jeweils unabhängig voneinander Methyl, Ethyl, n-Propyl, iso-Propyl, n-Butyl, sec.-Butyl oder tert.-Butyl bedeutet, wobei mindestens eine, nicht direkt mit N oder P verbundenen, CH₂-Gruppe der genannten Reste R durch O ersetzt sein kann,
Y CH₂, O, S oder NR' bedeutet und
R' Methyl, Ethyl, n-Propyl, iso-Propyl, n-Butyl, sec.-Butyl oder tert.-Butyl bedeutet,
zunächst mit einer Verbindung der Formel (III) umgesetzt wird,

   Hal-(CH₂)-(CH₂)ᵤ-OH (III),

   wobei u 1, 2, 3, 4, 5, 6 oder 7 bedeutet, wobei mindestens eine nicht an den Sauerstoff angebundene CH₂-Gruppe der -(CH₂)ᵤ-Alkylenkette mit u = 3, 4, 5, 6 oder 7 durch O ersetzt sein kann oder mindestens eine Doppelbindung enthalten kann, und Hal Cl, Br oder I bedeutet, und die entstehende Zwischenverbindung der Formel (IV)

   [K-(CH₂)-(CH₂)ᵤ-OH]⁺ [Hal]- (IV),

   wobei K, u und Hal eine der zuvor genannten Bedeutungen haben,
   mit einer Verbindung der Formel (V)

   R"-(CH₂)ᵥ-SO₂-L (V),

   wobei v und R" eine zuvor genannte Bedeutung haben und L eine Abgangsgruppe ausgewählt aus einer geradkettigen oder verzweigten Alkoxygruppe mit 1 bis 3 C-Atomen, Cl oder F bedeutet, vorzugsweise Cl oder F bedeutet,
   unter Säurekatalyse im Fall der Alkoxygruppe als Abgangsgruppe oder in Gegenwart einer Base im Fall von Cl oder F als Abgangsgruppe, umgesetzt wird und die entstehende Verbindung der Formel (VI)

   [K-(CH₂)-(CH₂)ᵤ-SO₃-(CH₂)ᵥ-R"]⁺ [Hal]- (VI),

   in der K, u, v, R" und Hal eine der zuvor genannten Bedeutungen haben,
   in einer Metathesereaktion mit einer Verbindung der Formel (VII) umgesetzt wird,

   [Kt]⁺[A]⁻ (VII),

   wobei [Kt]⁺ ein Alkalimetallkation oder H⁺ bedeutet und [A]- eine für die Anionen der Formel I beschriebene Bedeutung hat.

Sowohl die Verbindungen der Formel II als auch die Verbindungen der Formel III, wie zuvor beschrieben, sind in der Regel im Handel erhältlich oder nach bekannten, zum Standard gehörenden Verfahren synthetisierbar.

Für die Synthese von Verbindungen der Formel (I), wie zuvor beschrieben oder als bevorzugt beschrieben, wobei SO₃ der Teilstruktur 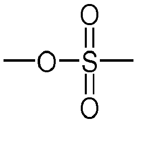 entspricht und in denen u 0 bedeutet, kann die Verbindung N-Oxymethylpyrrolidin der Formel (IIIa)

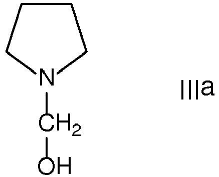

in dem zuvor beschriebenen Syntheseweg für die weitere Umsetzung benutzt werden. Die Verbindung der Formel (IIIa) kann nach literaturbekannten Verfahren hergestellt werden, beispielsweise wie in N.J. Putochin, Chem. Berichte, 55, 1922, S.2749-2753 beschrieben.

Die Umsetzung der Verbindungen der Formel II mit Verbindungen der Formel III, wie zuvor beschrieben, finden in der Regel bei Reaktionstemperaturen zwischen 10°C und 200°C, vorzugsweise zwischen 20°C und 100°C statt. In der Regel wird kein Lösungsmittel verwendet.

Die Zwischenverbindungen der Formel (IV)

[K-(CH₂)-(CH₂)ᵤ-OH]⁺ [Hal]- (IV),

wobei K, u und Hal eine der zuvor genannten Bedeutungen haben, werden in der Regel durch Extraktion oder Fällung aus dem Reaktionsgemisch der Umsetzung erhalten. Eine Aufreinigung nach herkömmlichen Methoden, wie beispielsweise der Umkristallisation, ist möglich. Vorzugsweise wird ohne weitere Aufreinigung der Verbindungen weiter umgesetzt.

Die Umsetzung der Verbindungen der Formel (IV), wie zuvor beschrieben, mit Verbindungen der Formel (V)

R"-(CH₂)ᵥ-SO₂-L (V),

wobei v und R" eine zuvor genannte Bedeutung haben und L eine Abgangsgruppe ausgewählt aus einer geradkettigen oder verzweigten Alkoxygruppe mit 1 bis 3 C-Atomen, Cl oder F bedeutet, vorzugsweise Cl oder F bedeutet, entsprechen einer klassischen Umesterung oder Substitutionsreaktion, deren Reaktionsbedingungen dem Fachmann auf dem Gebiet der organischen Synthese bekannt ist.

Ist die Abgangsgruppe L in der Verbindung der Formel V eine Alkoxygruppe, wie zuvor beschrieben, so findet die Umsetzung unter Säurekatalyse statt, beispielsweise in Gegenwart von Schwefelsäure, Toluolsulfonsäure, Methan- oder Ethan-sulfonsäure, Nafion®, Amberlite® oder Amberlyst® in saurer Form.

Ist die Abgangsgruppe L in der Verbindung der Formel V Cl oder F, so findet die Umsetzung in Gegenwart einer Base statt, beispielweise anorganischen Basen wie KOH, NaOH, Na₂CO₃, NaHCO₃ oder organischen Basen wie Triethylamin, Di-isopropyl-ethylamin oder Pyridin.

Diese Substitutionsreaktion findet bevorzugt in Gegenwart eines organischen Lösungsmittels statt. Die Zugabe der Verbindung der Formel V findet bei Temperaturen zwischen -10° C und Raumtemperatur statt. Um die Reaktion zu vervollständigen kann Erhitzen zum Rückfluss in einigen Fällen empfohlen werden.
Bei der Umesterung mit Substanzen Formel V, wie beschrieben, in denen die Abgangsgruppe L eine Alkoxygruppe, bevorzugt Methoxy bedeutet, liegt die übliche Zeit der Erwärmung in der Größenordnung von Stunden, mit Abdestillierung, um den gebildeten Alkohol (Methanol) zu entfernen.

Die aus der Substitutionsreaktion erhaltene Zwischenverbindung (VI)

[K-(CH₂)-(CH₂)ᵤ-SO₃-(CH₂)ᵥ-R"]⁺ [Hal]⁻ (VI),

in der K, u, v, R" und Hal eine der zuvor genannten Bedeutungen haben,
wird in einer Metathesereaktion mit einer Verbindung der Formel (VII) umgesetzt wird,

[Kt]⁺[A]⁻ (VII),

wobei [Kt]⁺ ein Alkalimetallkation oder H⁺ bedeutet und [A]- eine für die Anionen der Formel (I) beschriebene Bedeutung oder als bevorzugt beschriebene Bedeutung hat und es entstehen Verbindungen der Formel (I), in denen SO₃ der Teilstruktur 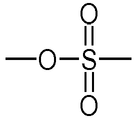 entspricht.

Die Metathesereaktion wird vorzugsweise in Wasser durchgeführt, wobei Temperaturen von 10°-100°C, vorzugsweise 15°-60°C, besonders bevorzugt Raumtemperatur, geeignet sind.

Alternativ kann die Reaktion jedoch auch in organischen Lösungsmitteln bei Temperaturen zwischen 10° und 100°C durchgeführt werden. Geeignete Lösungsmittel sind hier Acetonitril, Aceton, 1,4-Dioxan, Dichlormethan, Dimethoxyethan oder ein Alkohol, beispielsweise Methanol, Ethanol oder iso-Propanol.

Bevorzugt werden Natrium- oder Kaliumsalze der Verbindungen der Formel (VII) eingesetzt.

Ein weiterer Gegenstand der Erfindung ist ein Verfahren zur Herstellung von Verbindungen der Formel (I), wie zuvor beschrieben oder als bevorzugt beschrieben, wobei SO₃ der Teilstruktur 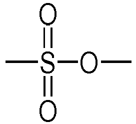 entspricht und u 2, 3, 4 oder 5 bedeutet, dadurch gekennzeichnet, dass eine Verbindung der Formel (II)

K-1 (II),

wobei K-1 ausgewählt wird aus der Gruppe R₃N, R₃P, 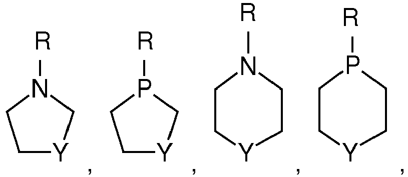 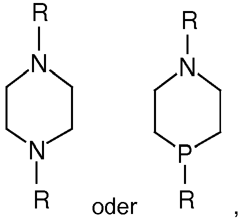 wobei R jeweils unabhängig voneinander Methyl, Ethyl, n-Propyl, iso-Propyl, n-Butyl, sec.-Butyl oder tert.-Butyl bedeutet, wobei mindestens eine, nicht direkt mit N oder P verbundene CH₂-Gruppe der genannten Reste R durch O ersetzt sein kann,
Y CH₂, O, S oder NR' bedeutet und
R'Methyl, Ethyl, n-Propyl, iso-Propyl, n-Butyl, sec.-Butyl oder tert.-Butyl bedeutet, zunächst mit einer Verbindung der Formel (VIII) 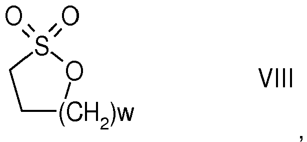 wobei w 1, 2, 3 oder 4 bedeutet,
   zu der Zwischenverbindung der Formel (IX)

   [K-(CH₂)-(CH₂)ᵤ-SO₃⁻] (IX),

   wobei K und u eine zuvor angegebene Bedeutung haben,
   umgesetzt wird, und anschließend die Zwischenverbindung der Formel IX, mit einem Alkylierungsmittel der Formel (X) umgesetzt wird

   L*-(CH₂)ᵥ-R" (X),

   wobei v und R" eine zuvor angegebene oder als bevorzugt angegebene Bedeutung haben und L* CF₃-S(O)₂O, C₄F₉-S(O)₂O, (C₂F₅)₂P(O)O, (C₄F₉)₂P(O)O, (Alkyl)₂O⁺, Alkyl-S(O)₂O, Alkyl-O-S(O)₂O, I oder Br bedeutet, wobei Alkyl jeweils unabhängig voneinander eine geradkettige oder verzweigte Alkylgruppe mit 1 bis 4 C-Atomen bedeutet,
   und die erhaltene Verbindung der Formel (XI)

   [K-(CH₂)-(CH₂)ᵤ-SO₃-(CH₂)ᵥ-R"]⁺ [L*]⁻ (XI),

   wobei K, u, v, R" und L* eine zuvor genannte Bedeutung haben,
   gegebenenfalls mit einer Verbindung der Formel (VII) umgesetzt wird,

   [Kt]⁺[A]⁻ (VII),

   wobei [Kt]⁺ ein Alkalimetallkation oder H⁺ bedeutet und [A]- eine für die Verbindungen der Formel I zuvor beschriebene Bedeutung hat, wenn das Anion [L*]⁻ nicht schon einer Bedeutung des Anions [A]- entspricht.

Sultone der der Formel (VIII) 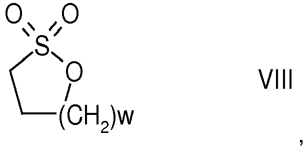 wobei w 1, 2, 3 oder 4 bedeutet, sind kommerziell erhältlich oder können nach bekannten Verfahren hergestellt werden, wie z.B. bei J.F. King et.al in Phosphorus and Sulfur and the Related Elements, 13 (1987), pp.161-175 beschrieben.

Die -(CH₂)-Cycloalkylenkette der Verbindung VIII kann auch mindestens eine Doppelbindung enthalten (A.L. Flohic et.al in Synlett, Nr. 5 (2003), pp. 667-670), jedoch nach allgemeinen Fachwissen nicht an den C-Atomen, die mit den Heteroatomen S und O eine direkte Bindung eingehen.

Die Umsetzung der kommerziell erhältlichen Verbindungen der Formel II, wie zuvor beschrieben mit den Sultonen der Formel VIII findet ohne Lösungsmittel oder in einem organischen Lösungsmittel, bevorzugt in einem organischen Lösungsmittel statt, beispielsweise in Toluol, Acetonitril, Dioxan, Aceton, Monoglyme, Diglyme oder in Gemischen von Acetonitril mit Dialkylether, besonders bevorzugt in Toluol statt. Die Reaktion findet bei Temperaturen zwischen -10° C und Raumtemperatur statt. Um die Reaktion zu vervollständigen, kann Erhitzen unter Rückfluss in einigen Fällen empfohlen werden.

Die aus diesem Verfahrensschritt erhaltene Zwischenverbindung der Formel (IX)

[K-(CH₂)-(CH₂)ᵤ-SO₃⁻] (IX),

wobei K und u eine der zuvor angegebenen Bedeutung haben, wird mit einem Alkylierungsmittel der Formel (X) umgesetzt

L*-(CH₂)ᵥ-R" (X),

wobei v und R" eine zuvor angegebene Bedeutung haben und L* CF₃-S(O)₂O, C₄F₉-S(O)₂O, (C₂F₅)₂P(O)O, (C₄F₉)₂P(O)O, (Alkyl)₂O⁺, Alkyl-S(O)₂O, Alkyl-O-S(O)₂O, I oder Br bedeutet, wobei Alkyl jeweils unabhängig voneinander eine geradkettige oder verzweigte Alkylgruppe mit 1 bis 4 C-Atomen bedeutet.

Alkylierungsmittel der Formel X sind kommerziell erhältlich oder können nach bekannten Verfahren hergestellt werden, wie z.B. CF₃SO₂O-CH=CH₂ (P.J. Stang und J. Ullmann, Angew. Chem., 103 (1991), S. 1549-1550) oder CH₃SO₂O-CH₂CH=C (R.F. Hudson and R.J. Withey, J. Chem. Soc. (B), 1966, pp. 237-240).

Die Alkylierung kann ohne Lösungsmittel durchgeführt werden, wobei Temperaturen von 0°-150°C, vorzugsweise 0°-80°C, besonders bevorzugt Raumtemperatur, geeignet sind. Alternativ findet die Reaktion jedoch auch in Gegenwart eines organischen Lösungsmittels statt, beispielsweise Acetonitril, Propionitril, Benzonitril, Dialkylether, Dichlormethan, Monoglyme, Diglyme, besonders bevorzugt in Acetonitril statt.
Die Reaktionstemperatur liegt zwischen 0°C und 150°C, bevorzugt zwischen 0°C und 80°C. Besonders bevorzugt wird bei Raumtemperatur umgesetzt.

Die aus dieser Alkylierung entstehenden Verbindungen der Formel (XI),

[K-(CH₂)-(CH₂)ᵤ-SO₃-(CH₂)ᵥ-R"]⁺ [L*]⁻ (XI),

wobei K, u, v, R" und L* eine zuvor genannte Bedeutung haben, können je nach Wahl der Abgangsgruppe L* schon eine Verbindung der Formel I darstellen oder es wird in der klassischen Metathesereaktion das gewünschte Anion ausgetauscht, in dem mit einer Verbindung der Formel VI umgesetzt wird,

[Kt]⁺[A]⁻ (VII),

wobei [Kt]⁺ ein Alkalimetallkation oder H⁺ bedeutet und [A]⁻ eine zuvor genannte Bedeutung hat. Für die Metathesereaktion gelten die Ausführungen wie zuvor beschrieben.

Alternativ kann die Zwischenverbindung der Formel IX zuerst mit einer Säure [H]⁺[A]⁻ umgesetzt werden, wobei A eine zuvor angegebenen Bedeutung hat, und in einem weiterem Schritt mit einer Diazoverbindung der Formel N₂-(CH₂)ᵥ-R" direkt zu Verbindung der Formel I alkyliert werden, wobei v und R" eine zuvor angegebene Bedeutung haben.

Ein weiterer Gegenstand der Erfindung ist ein Verfahren zur Herstellung von Verbindungen der Formel I, wie zuvor beschrieben oder als bevorzugt beschrieben, wobei SO₃ der Teilstruktur 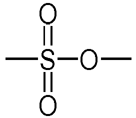 entspricht, dadurch gekennzeichnet, dass eine Verbindung der Formel (II)

K-1 (II),

wobei K-1 ausgewählt wird aus der Gruppe R₃N, R₃P, 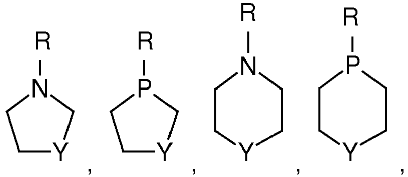 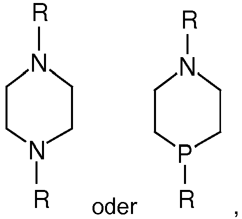 wobei
R jeweils unabhängig voneinander Methyl, Ethyl, n-Propyl, iso-Propyl, n-Butyl, sec.-Butyl oder tert.-Butyl bedeutet, wobei einzelne, nicht direkt mit N oder P verbundene CH₂-Gruppen durch O ersetzt sein können,
Y CH₂, O, S oder NR' bedeutet und
R'Methyl, Ethyl, n-Propyl, iso-Propyl, n-Butyl, sec.-Butyl oder tert.-Butyl bedeutet,
   zunächst mit einer Verbindung der Formel (XII) umgesetzt wird,

   Hal-(CH₂)-(CH₂)ᵤ-Hal (XII),

   wobei u 0, 1, 2, 3, 4, 5, 6 oder 7 bedeutet, wobei mindestens eine nicht an Hal angebundene CH₂-Gruppe der -(CH₂)ᵤ-Alkylenkette mit u = 2, 3, 4, 5, 6 oder 7 durch O ersetzt sein kann oder mindestens eine Doppelbindung enthalten kann und Hal Cl, Br oder I bedeutet, und die entstehende Zwischenverbindung der Formel (XIII)

   [K-(CH₂)-(CH₂)ᵤ-Hal]⁺ [Hal]- (XIII),

   wobei K, u und Hal eine der zuvor genannten Bedeutungen haben,
   mit einem Alkalimetallsulfit oder Ammoniumsulfit zu einer Zwischenverbindung der Formel IX umsetzt

   [K-(CH₂)-(CH₂)ᵤ-SO₃⁻] (IX),

   wobei K und u eine der zuvor angegebenen Bedeutung haben und anschließend mit einem Alkylierungsmittel der Formel (X) alkyliert,

   L*-(CH₂)ᵥ-R" (X),

   wobei v und R" eine zuvor angegebene Bedeutung haben und L* CF₃-S(O)₂O, C₄F₉-S(O)₂O, (C₂F₅)₂P(O)O, (C₄F₉)₂P(O)O, (Alkyl)₂O⁺, Alkyl-S(O)₂O, Alkyl-O-S(O)₂O, I oder Br bedeutet, wobei Alkyl jeweils unabhängig voneinander eine geradkettige oder verzweigte Alkylgruppe mit 1 bis 4 C-Atomen bedeutet.

Alternativ kann man eine Verbindung der Formel (II), wie zuvor beschrieben, zuerst mit Hal-(CH₂)-(CH₂)ᵤ-S(O)₂ONa der Formel (Xlla) (Hal ist vorzugsweise Br oder I und u hat eine der zuvor genannten Bedeutungen) zu einer Zwischenverbindung der Formel (IX) alkylieren, wie zuvor beschrieben, und weiter mit einem Alkylierungsmittel der Formel (X) umsetzen, wie zuvor beschrieben. Die Herstellungsmethode für die Verbindungen der Formel (Xlla) (u = 0 und 1) ist bei Feng Gao et al. in Bioorganic & Medicinal Chemistry Letters 18 (2008), pp. 5518-5522, beschrieben.

Die aus Alkylierungsreaktion entstehenden Verbindungen der Formel (XI),

[K-(CH₂)-(CH₂)ᵤ-SO₃-(CH₂)ᵥ-R"]⁺ [L*]⁻ (XI),

wobei K, u, v, R" und L* eine zuvor genannte Bedeutung haben, können je nach Wahl der Abgangsgruppe L* schon eine Verbindung der Formel I darstellen oder es wird in der klassischen Metathesereaktion das gewünschte Anion ausgetauscht, in dem mit einer Verbindung der Formel VI umgesetzt wird,

[Kt]⁺[A]⁻ (VII),

wobei [Kt]⁺ ein Alkalimetallkation oder H⁺ bedeutet und [A]- eine zuvor genannte Bedeutung hat. Für die Metathesereaktion gelten die Ausführungen wie zuvor beschrieben. Verbindungen der Formel (XII), wie zuvor beschrieben,

Hal-(CH₂)-(CH₂)ᵤ-Hal (XII),

sind kommerziell erhältlich , wobei u 0, 1, 2, 3, 4, 5, 6 oder 7 bedeutet und wobei mindestens eine nicht an Hal angebundene CH₂-Gruppe der -(CH₂)ᵤ-Alkylenkette mit u = 2, 3, 4, 5, 6 oder 7 durch O ersetzt sein kann oder mindestens eine Doppelbindung enthalten kann, oder können aus den kommerziell erhältlichen Verbindungen der Formel HO-(CH₂)-(CH₂)ᵤ-OH, wobei u 1, 2, 3, 4, 5, 6 oder 7 bedeutet und wobei mindestens eine nicht an den Sauerstoff angebundene CH₂-Gruppe der -(CH₂)ᵤ-Alkylenkette mit u = 3, 4, 5, 6 oder 7 durch O ersetzt sein kann oder mindestens eine Doppelbindung enthalten kann, hergestellt werden. Die Reaktionsbedingungen derartiger Substitutionen sind dem Fachmann auf dem Gebiet der organischen Synthese hinlänglich bekannt.

Die entstehende Zwischenverbindung der Formel (XIII)

[K-(CH₂)-(CH₂)ᵤ-Hal]⁺ [Hal]- (XIII),

wobei K, u und Hal eine der zuvor genannten Bedeutungen haben,
wird mit einem Alkalimetallsulfit oder Ammoniumsulfit und anschließend mit einer Verbindung der Formel (X) umgesetzt.

L*-(CH₂)ᵥ-R" (X),

wie zuvor beschrieben.

Die Reaktionsbedingungen für derartige Umsetzungen mit Verbindungen der Formel (X) und der sich anschließenden Metathesereaktion wurden zuvor beschrieben und gelten auch in diesem Verfahren entsprechend.

Ein weiterer Gegenstand ist ein Elektrolyt enthaltend mindestens eine Verbindung der Formel (I), wie zuvor beschrieben, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, oder als bevorzugt beschrieben.

Chemisch gesehen ist ein Elektrolyt eine beliebige Substanz, die freie Ionen enthält und dadurch elektrisch leitfähig ist. Das typischste Elektrolyt ist eine ionische Lösung, aber Schmelz- und Festelektrolyte sind ebenfalls möglich.
Ein erfindungsgemäßer Elektrolyt oder eine entsprechende Elektrolytformulierung ist daher ein elektrisch leitfähiges Medium, hauptsächlich aufgrund der Anwesenheit mindestens einer Substanz, die in gelöstem und/oder in geschmolzenem Zustand vorliegt, d.h. eine elektrische Leitfähigkeit durch Bewegung von lonenspezies unterstützt.

Die Verbindungen der Formel I, wie zuvor beschrieben, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, oder als bevorzugt beschrieben, übernehmen in dem Elektrolyten die Funktion eines Additivs.

Für den Einsatz in Lithium-Batterien, Lithium-Ionen-Batterien oder Lithium-Kondensatoren übernehmen die Verbindungen der Formel I, wie zuvor beschrieben, wobei [A]⁻ auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, die Funktion eines Additivs, die insbesondere positiv auf die Erzeugung der SEI einwirken.

Für den Einsatz in Solarzellen, elektrochromatischen Vorrichtungen, Sensoren oder Biosensoren übernehmen die Verbindungen der Formel I, wie zuvor beschrieben, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, die Funktion eines Additivs.

Werden die Verbindungen der Formel I, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, demzufolge als Additiv in den erfindungsgemäßen Elektrolyten eingesetzt, so liegt die typische Konzentration zwischen 0,05 und 10 Gewichtsprozent, vorzugsweise zwischen 0,05 und 5 %, bezogen auf das Gesamtgewicht des Elektrolyten.

Für die Zwecke der vorliegenden Erfindung bezieht sich die Molarität auf die Konzentration bei 25°C.

Ein weiterer Gegenstand der Erfindung ist daher die Verwendung von Verbindungen der Formel (I), wie zuvor beschrieben, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, als Additiv in Elektrolyten.

Bevorzugt werden die erfindungsgemäßen Verbindungen der Formel (I), wie zuvor beschrieben, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, in Lithium-Batterien oder Lithium-Ionen-Batterien verwendet bzw. werden bevorzugt in Elektrolyten eingesetzt, die für diese elektrochemischen Vorrichtungen geeignet sind.

Bevorzugt enthält der erfindungsgemäße Elektrolyt daher neben den Verbindungen der Formel (I), wie zuvor beschrieben oder bevorzugt beschrieben, wobei [A]⁻ auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, ein Leitsalz, bevorzugt ausgewählt aus einem Lithiumsalz und/oder einem Tetraalkylammoniumsalz, wobei die Alkylgruppen jeweils unabhängig voneinander eine Alkylgruppe mit 1 bis 4 C-Atomen bedeutet.

In einer bevorzugten Ausführungsform bei Verwendung des Elektrolyten in Lithium- oder Lithium-lonen-Batterien und Lithium-Ionen Kondensatoren, ist das Leitsalz ein Lithiumleitsalz wie LiPF₆, LiBF₄, LiN(SO₂F)₂, LiN(SO_{2C}F₃)₂, LiN(SO₂C₂F₅)₂, LiF₅P(C₂F₅), LiF₅P(C₃F₇), LiF₅P(C₄F₉), LiF₃P(C₂F₅)₃, LiF₃P(C₄F₉)₃, LiB(C₂O₄) oder LiF₂B(C₂O₄)₂.

In einer bevorzugten Ausführungsform bei Verwendung des Elektrolyten in Doppelschicht- oder Superkondensatoren, ist das Leitsalz ein Tetraalkylammoniumsalz aus der Gruppe [N(C₂H₅)₄]BF₄, [N(C₂H₅)₄]PF₆, [N(C₂H₅)₃(CH₃)]BF₄, [N(C₂H₅)₃(CH₃)]PF₆, [N(C₂H₅)₄][N(SO₂CF₃)₂], [N(C₂H₅)₃(CH₃)][N(SO₂CF₃)₂], [N(C₂H₅)₄][PF₃(C₂F₅)₃], [N(C₂H₅)₃(CH₃)][PF₃(C₂F₅)₃], [N(C₂H₅)₄][PF₄(C₂F₅)₂], [N(C₂H₅)₃(CH₃)][PF₄(C₂F₅)₂]. [N(C₂H₅)₄][PF₅(C₂F₅)], [N(C₂H₅)₃(CH₃)][PF₅(C₂F₅)].

Bevorzugt werden 0,45 bis 2 molare, besonders bevorzugt 1 molare Lösungen des Lithium-Leitsalzes in einem aprotischen Lösungsmittel oder Lösungsmittelgemisch verwendet.

Die erfindungsgemäßen Elektrolyte enthalten vorzugsweise ein aprotisches Lösungsmittel oder Lösungsmittelgemisch, sowie gegebenenfalls ein oder mehr weitere Additive. Sie können in Kombination mit weiteren Leitsalzen und/oder Zusatzstoffen, als Bestandteil eines Polymerelektrolyts oder Phasentransfermediums verwendet werden.

Vorzugsweise besteht das aprotische Lösungsmittel des Elektrolyten aus organischen offenkettigen oder zyklischen Carbonaten, Carbonsäureestern, Nitrilen, Silanen oder Sulfonsäureestern oder einem Gemisch daraus. Nitrile, insbesondere Acetonitril, werden als Lösungsmittel vorzugsweise in Doppelschichtkondensatoren eingesetzt.

Bevorzugte offenkettige oder zyklische Carbonate sind Diethylcarbonat, Dimethylcarbonat, Ethylmethylcarbonat, Ethylencarbonat oder Propylencarbonat.
Bevorzugte Carbonsäureester sind Ethylacetat oder Methylpropionat.
Bevorzugte Nitrile sind Adipinsäuredinitril, Valeronitril und Acetonitril, besonders bevorzugt ist Acetonitril.
Das organische Lösungsmittel ist vorzugsweise in 5 bis 90 Gewichtsprozent, vorzugsweise in 40 bis 90 Gewichtsprozent in dem Elektrolyt enthalten, wobei sich die Gewichtsprozentangabe auf den gesamten Elektrolyten bezieht.

Weitere Additive können beispielsweise aus den bekannten Additiven Vinylencarbonat, Propansulton, Vinylacetat, Biphenyl, Cyclohexylbenzen, organischen Aminen, beispielsweise Trialkylaminen, Dialkylphenylaminen oder N-silylierte Amine, wie Trimethylsilylimidazol als Beispiel für ein N-silyliertes cyclisches Amin, oder verschiedenen Sulphonen, beispielsweise Diphenylsulphon, ausgewählt werden, wobei die Alkylgruppen in den erwähnten Aminen jeweils unabhängig voneinander eine geradkettige oder verzweigte Alkylgruppe mit 1 bis 20 C-Atomen sein können.

In einer bevorzugten Ausführungsform sind neben den erfindungsgemäßen Additiven der Formel I, wie zuvor beschrieben, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, auch die Additive der genannten Gruppe Vinylencarbonat, Propansulton, Vinylacetat, Biphenyl, Cyclohexylbenzen, organische Amine, N-silylierte Amine oder Sulphone, wobei die Alkylgruppen in den erwähnten Aminen jeweils unabhängig voneinander eine geradkettige oder verzweigte Alkylgruppe mit 1 bis 20 C-Atomen sein können, in dem Elektrolyten enthalten.

Eine andere Klasse von Additiven, die enthalten sein können, sind Additive, die eine Gelbildung hervorrufen für sogenannte Gel-Elektrolyte, das sind Elektrolyte, die einen "quasi-soliden" Zustand einnehmen. Sie haben strukturelle Eigenschaften von Festelektrolyten, behalten aber leitfähige Eigenschaften wie Flüssigelektrolyte.
Derartige Gel-Additive können von anorganischen partikulären Materialien ausgewählt werden, beispielsweise SiO₂, TiO₂ oder Al₂O₃. Die erfindungsgemäßen Elektrolyte können derartige Gel-Additive in 0,01 bis 20 Gewichtsprozent enthalten, bezogen auf den Gesamt-Elektrolyten, vorzugsweise in 1 bis 10 Gewichtsprozent.

Bei Vorhandensein eines Lösungsmittels in dem erfindungsgemäßen Elektrolyten kann weiterhin ein Polymer enthalten sein, wobei es sich bei dem Polymer um Polyvinylidenfluorid, Polyvinyliden-Hexafluorpropylen-, Polyvinyliden-Hexafluorpropylen-Chlortrifluorethylen-Copolymere, Nafion, Polyethylenoxid, Polymethylmethacrylat, Polyacrylnitril, Polypropylen, Polystyrol, Polybutadien, Polyethylenglycol, Polyvinylpyrrolidon, Polyanilin, Polypyrrol, Polythiophen handelt. Diese Polymere werden den Elektrolyten zugesetzt, um Flüssigelektrolyte in quasi-feste oder Festelektrolyte umzuwandeln und so die Lösungsmittelretention zu verbessern, vor allem beim Altern.

Die Herstellung der erfindungsgemäßen Elektrolyte erfolgt nach Methoden, die dem Fachmann auf dem Gebiet der Herstellung von Elektrolyten bekannt ist, in der Regel durch Lösen des Leitsalzes in dem entsprechenden Lösungsmittelgemisch und Zugabe der erfindungsgemäßen Additive der Formel I, wie zuvor beschrieben, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4.
Ein weiterer Gegenstand der Erfindung ist eine elektrochemische oder elektrooptische Vorrichtung enthaltend mindestens eine Verbindung der Formel I, wie zuvor beschrieben oder als bevorzugt beschrieben.

Vorzugsweise kann die Vorrichtung eine Lithium-Batterie, eine Lithium-Ionen-Batterie, ein Doppelschichtkondensator, ein Lithium-Ionen-Kondensator, eine Solarzelle, ein elektrochromes Display, ein Sensor oder ein Biosensor sein.

Bevorzugt ist die Solarzelle eine Farbstoffsolarzelle.
Ein weiterer Gegenstand der Erfindung ist die Verwendung einer Verbindung der Formel I, wie zuvor beschrieben oder als bevorzugt beschrieben in elektrochemischen oder elektrooptischen Vorrichtungen, wie zuvor beschrieben.

Eine Lithium-Batterie ist eine Batterie, in der als negative Elektrode eine Lithium-Metall-Elektrode verwendet wird
Eine Lithium-Ionen-Batterie verwendet als negative Elektrode Materialien, in die Lithium-Ionen reversibel ein- und ausgelagert werden können. Beispiele hierfür sind Graphit, Silizium oder Silizium -Kohlenstoff-Komposite, Zinnoxide oder Lithiumtitanoxide.

Der generelle Aufbau derartiger elektrochemischer und elektrooptischer Vorrichtungen ist bekannt und dem Fachmann auf diesem Gebiet geläufig, beispielsweise für Batterien in Linden's Handbook of Batteries (ISBN 978-0-07-162421-3).

Beispielsweise besteht die Anode aus Kohlenstoff/Graphit, die Kathode aus einem Lithium-Metalloxid oder Lithiumphosphat und der Separator aus Polypropylen/Polyethylen oder keramischer Folie.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die synthetisierten Verbindungen werden durch NMR-Spektroskopie charakterisiert. Die NMR-Proben werden in 5mm NMR-Röhrchen bei 25°C in einem Bruker Avance III Spektrometer, ausgestattet mit einem 9.3980 T Kryomagneten, vermessen. Die ¹H und ¹⁹F NMR-Spektren werden gemessen, in dem ein 5 mm Kombinations-H/¹⁹F-Messkopf verwendet wird, der bei 400.17 und 376.54 MHz arbeitet. Die ¹³C NMR-Spektren werden erhalten, in dem ein 5mm Breitband-Invers-Messkopf bei 100.62 MHz verwendet wird. Die Referenz bei den ¹H NMR-Spektren bildet Tetramethylsilan (TMS) unter Benutzung der chemischen Verschiebungen der Lösungsmittel CHCl₃ (7.23 ppm) und CH₃CN (1.96 ppm). Entsprechendes gilt für die¹³C NMR-Spektren:CHCl₃ (77.24 ppm) und CH₃CN (118.70 ppm).

### Beispiele:

### Beispiel 1. Synthese von 3-(1-Methylpyrrolidinium-1-yl)propan-1-sulfonat

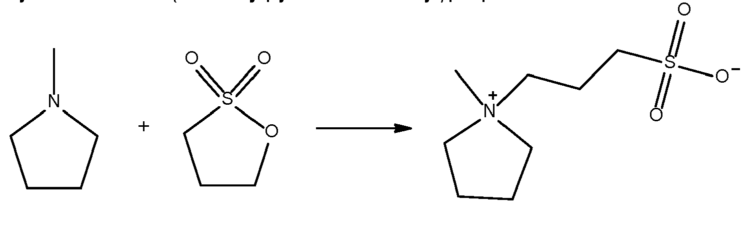

Zu 23,8 g (195 mmol) 1,3-Propansulton in 50 mL Toluol werden unter Kühlung im Eis-Bad 16,8 g (197 mmol) Methylpyrrolidin langsam zugetropft. Der Niederschlag wird filtriert, zwei Mal mit Toluol gewaschen und im Vakuum (10⁻³ hPa) getrocknet. Man erhält 37,1 g 3-(1-Methylpyrrolidinium-1-yl)-propan-1-sulfonat als hygroskopischen, farblosen Feststoff. Die Ausbeute ist 92 %.
¹H-NMR (Lösungsmittel: D₂O), δ (ppm): 3,98(m, 6H); 3,55 (s, 3H); 3,42 (t, J = 7,2 Hz, 2H); 2,74 (m, 6H).
¹³C{¹H}-NMR (Lösungsmittel: D₂O), δ (ppm): 64,5 s; 62,4 s; 48,1s; 47,5 s; 21,3 s; 19,3 s.

### Beispiel 2. Synthese von 1-(3-(Methoxysulfonyl)propyl)-1- methylpyrrolidiniumtris(pentafluorethyl)trifluorophosphat

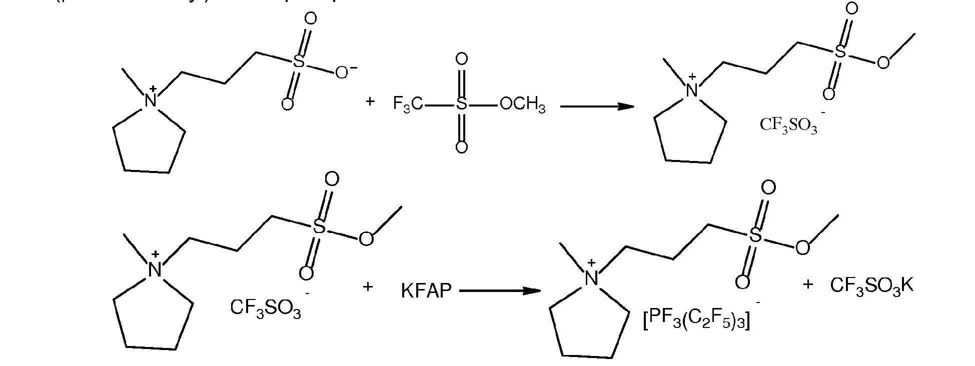

Es werden 6,3 g (30,3 mmol) 3-(1-methylpyrrolidinium-1-yl)propan-1-sulfonat in 10 mL Acetonitril suspendiert und mit 5,6 g (34,0 mmol) Methyltriflat versetzt. Der Feststoff löst sich in einer exothermen Reaktion vollständig auf und die Reaktionsmischung wird für drei Stunden bei Raumtemperatur gerührt. Bei Zugabe von 14,7 g (30,3 mmol) Kalium Tris(pentafluorethyl)trifluorophosphat (KFAP) in 50 mL eiskaltem Wasser bildet sich ein grobkörniger Niederschlag. Der Feststoff wird isoliert, vier Mal mit je 40 mL Wasser gewaschen und im Exsikkator getrocknet. Man erhält 19,2 g 1-(3-(Methoxysulfonyl)propyl)-1-methylpyrrolidinium-tris(pentafluorethyl)trifluorophosphat als schwach gelben Feststoff. Die Ausbeute ist 95 %.
¹H-NMR (Lösungsmittel: CD₃CN), δ (ppm): 3,91 (s, 3H); 3,44 (m, 4H); 3,36 (m, 2H); 3,24 (t, J = 7,4 Hz, 2H); 2,97 (s, 3H); 2,25 (m, 2H); 2,19 (m, 4H).
¹³C{¹H}-NMR (Kation) (Lösungsmittel: CD₃CN), δ (ppm): 64,7; 61,6; 56,7; 48,2; 45,2; 21,2; 18,5.

Elementaranalyse; berechnet (gefunden), %: C 27,00 (27,22), H 3,02 (3,02), N 2,10 (2,07), S 4,80 (4,85)

### Beispiel 3. Synthese von 1-(2-Hydroxyethyl)-1-methylpyrrolidinium-chlorid

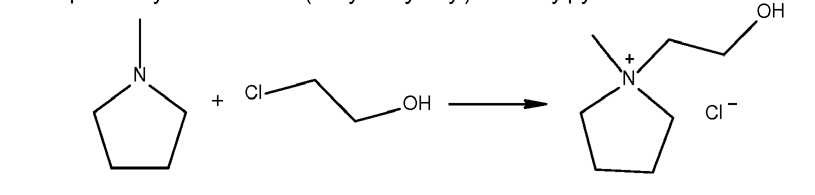

Es werden 14,8 g (174 mmol) Methylpyrrolidin in 20 mL Chlorethanol für 7 Stunden auf 70 °C erwärmt. Die klare, leicht gelbe Lösung wird mit 25 mL Diethylether versetzt wodurch das Produkt ausfällt. Der Niederschlag wird filtriert, mit Diethylether gewaschen und man erhält 28 g 1-(2-Hydroxyethyl)-1-methylpyrrolidinium-chlorid als leicht gelben, wachsartigen Feststoff. Die Ausbeute ist 97 %.
¹H-NMR (Lösungsmittel: CD₃CN), δ (ppm): 6,05 (t, J = 5,7 Hz, 1H); 3,91 (m, 2H); 3,62 (m, 4H); 3,52 (m, 2H); 3,15 (s, 3H); 2,14 (m, 4H).
¹³C{¹H}-NMR (Lösungsmittel: CD₃CN), δ (ppm): 66,6 s; 66,2 s; 56,9 s; 49,5 s; 22,3 s.

### Beispiel 4. Synthese von 1-Methyl-1-(2-((methylsulfonyl)oxy)ethyl)-pyrrolidinium-chlorid

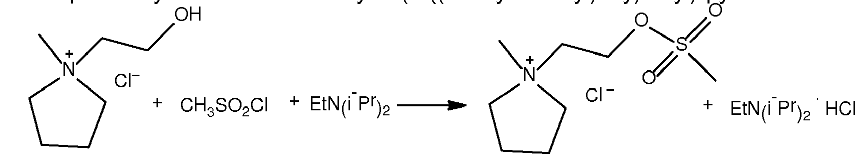

Es werden 5,3 g (32 mmol) 1-(2-hydroxyethyl)-1-methylpyrrolidinium-chlorid in 25 mL Dichlormethan aufgenommen und mit 4,6 g (35,7 mmol) Di-isopropyl-ethylamin versetzt. Anschließend werden bei Kühlung im Eisbad langsam 5 g (43 mmol) Methansulfonsäurechlorid zugetropft, wobei die Temperatur unter 15 °C gehalten wird. Das Produkt fällt als farbloser Feststoff aus und wird nach vier Stunden Reaktionszeit durch Filtration isoliert. Man erhält 5,2 g 1-Methyl-1-(2-(methylsulfoxy)ethyl)pyrrolidinium-chlorid. Die Ausbeute ist 66 %.
¹H-NMR (Lösungsmittel: DMSO-D₆), δ (ppm): 4,75 (m, 2H); 3,94 (m, 2H); 3,61 (m, 4H); 3,38 (s, 3H); 3,13 (s, 3H); 2,09 (m, 4H).
¹³C{¹H}-NMR (Lösungsmittel: DMSO-D₆), δ (ppm): 64,9 s; 64,6 s; 61,7 s;, 48,2 s; 37,7 s; 21,3 s.

### Beispiel 5. Synthese von 1-Methyl-1-(2-((methylsulfonyl)oxy)ethyl)-pyrrolidinium-tris(pentafluorethyl)trifluorophosphat

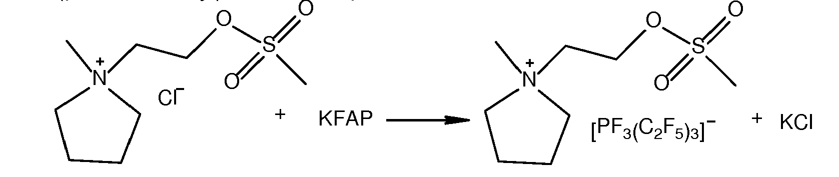

Man löst 5,2 g (21 mmol) 1-methyl-1-(2-(methylsulfoxy)ethyl)pyrrolidinium-chlorid in 20 mL eiskaltem Wasser auf und gibt 9,7 g (20 mmol) KFAP als wässrige Lösung zu. Der entstehende Niederschlag wird filtriert, fünf Mal mit je 30 mL eiskaltem Wasser gewaschen und im Exsikkator getrocknet. Man erhält 12,6 g 1-Methyl-1-(2-(methylsulfonyloxy)ethyl)-pyrrolidinium-tris(pentafluorethyl)trifluorophosphat als schwach rosa gefärbten Feststoff. Die Ausbeute ist 96 %.
¹H-NMR (Lösungsmittel: CD₃CN), δ (ppm): 4,59 (m, 2H); 3,68 (m, 2H); 3,52 (m, 4H); 3,13 (s, 3H); 3,03 (s, 3H); 2,19 (m, 4H).
¹³C{¹H}-NMR (Kation) (Lösungsmittel: CD₃CN), δ (ppm): 66,9 s; 64,7 s; 63,8 s; 50,0 s; 38,3 s; 22,4 s.

### Beispiel 6. Synthese von 1-Methyl-1-(2-((methylsulfonyl)oxy)ethyl)- pyrrolidinium-bis(oxalatoborat)

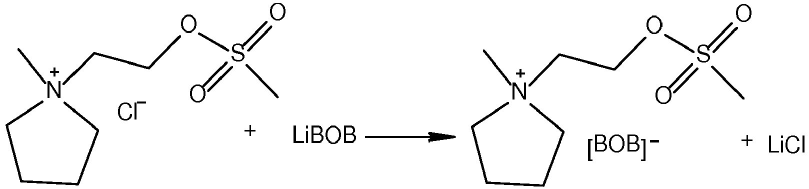

Die Synthese erfolgt analog zu Beispiel 5 durch Umsetzung mit Lithium-bis(oxalato)borat. Ausbeute ist 86 %.
¹H-NMR (Lösungsmittel: DMSO-D₆), δ (ppm): 4,68 (m, 2H); 3,81 (m, 2H); 3,54 (m, 4H); 3,31 (s, 3H); 3,06 (s, 3H); 2,11 (m, 4H).
¹¹B-NMR (Lösungsmittel: DMSO-d₆) δ (ppm): 7,4 (s),

### Beispiel 7. Alternative Synthese von 3-(1-Methylpyrrolidinium-1-yl)propan-1-sulfonat

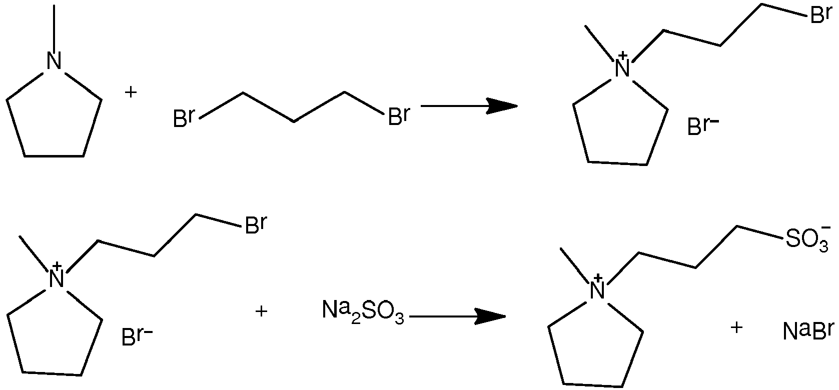

Es werden 16,1 g (79,6 mmol) 1,3-Dibrompropan in 25 mL THF gelöst und mit 6,0 g (70,6 mmol) Methylpyrrolidin versetzt. Bereits gegen Ende der Zugabe bildet sich ein fein verteilter farbloser Niederschlag. Die Mischung wird für 25 Stunden bei Raumtemperatur gerührt und der farblose Niederschlag wird durch Filtration isoliert. Man erhält 17,2 g 1-(3-brompropyl)-1-methylpyrrolidinium-bromid als farblosen, hygroskopischen Feststoff. Ausbeute 85 %.
¹H-NMR (Lösungsmittel: CD₃CN), δ (ppm): 3,59 (m, 8H), 3,09 (s, 3H), 2,35 (m, 2H), 2.16 (m, 4H).
¹³C{¹H}-NMR (Lösungsmittel:CD₃CN), δ (ppm): 65,8, 63,6, 49,7, 30,9, 28,2, 22,7.

Eine wässrige Lösung von 1-(3-brompropyl)-1-methylpyrrolidinium-bromid (1,9 g; 6,5 mmol) wird mit 1,0 g Natriumsulfit (8,3 mmol) versetzt und für drei Stunden auf 85 °C erwärmt. Das Lösemittel wird im Vakuum entfernt und man erhält 3-(1-methylpyrrolidinium)propan-1-sulfonat als farblosen Feststoff. Der Umsatz ist quantitativ.
NMR entspricht dem Produkt aus der Reaktion von Methylpyrrolidin mit 1,3-Propansulton (Beispiel 1).

### Beispiel 8. Synthese von 1-(Brommethyl)-1-methylpyrrolidinium-bromid

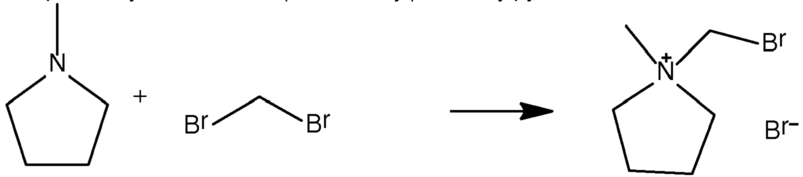

Es werden 21.5 g (123.7 mmol) Dibrommethan in 20 mL THF gelöst und mit 9.6 g (112.7 mmol) Methylpyrrolidin versetzt. Bereits gegen Ende der Zugabe bildet sich ein fein verteilter farbloser Niederschlag. Die Mischung wird für 16 Stunden bei Raumtemperatur gerührt und der farblose Niederschlag wird durch Filtration isoliert und mit THF gewaschen. Man erhält 4.8 g 1-(Brommethyl)-1-methylpyrrolidinium-bromid als farblosen, hygroskopischen Feststoff. Ausbeute 16 %.
Durch längere Reaktionszeit wird die Ausbeute erhöht. Aus ruhender Lösung fällt das Produkt in Form feiner Nadeln an.
¹H NMR (Lösungsmittel: DMSO-D₆), δ (ppm): 5.56 (s, 2H), 3.68 (m, 4H), 3.21 (s, 3H), 2.15 (m, 4H).
¹³C{¹H} NMR (Lösungsmittel: DMSO-D₆), δ (ppm): 63.9 s, 57.4 s, 48.7 s, 21.8 s.

### Elektrochemische Charakterisierung:

### Test Set-up

Als Testzellen werden Lithium-Ionen-Zellen bestehend aus einer Graphitelektrode, einem Polyolefin-basiertem Separator und einer LiNiMnCoO₂-Elektrode (Elektrodenfläche je 25 cm², Pouch-Cell-Aufbau) verwendet.

Vor dem Bau der Testzellen werden beide Elektroden bei 120 °C und Vakuum, sowie der Separator bei 50 °C ohne Vakuum min. 24 h getrocknet.

Testzellenbau:
- Die Anode wird mittig auf die kaschierte Aluminiumfolie gelegt, sodass der Ableiter ca. 20 mm über die Folie hinausragt.
- Die Kathode wird so auf die Anode gelegt, dass die Anode einen gleichmäßigen Rahmen um die Kathode bildet.
- Eine zweite kaschierte Aluminiumfolie wird so platziert, dass beide Folien deckungsgleich übereinander liegen. Zur Vermeidung von Kurzschlüssen wird jeweils zwischen Stromableiter und kaschierter Aluminiumfolie eine PE Folie gelegt.
- An der oberen Kante wird mit der Schweißzange (Temperatur 225°C) eine Schweißnaht setzen, sodass der Elektrodenstapel nicht mehr verrutschen kann.
- Unter Argon Schutzgasatmosphäre werden beide Elektroden mit jeweils 1ml des zu testenden Elektrolyten benetzt und ein Polyolefinseparator, der zuvor 5 min in eine mit Elektrolyt gefüllte Petrischale gelegt wurde, zwischen Anode und Kathode platziert.
- Linke und rechte Seite der Testzelle wird mit einer Schweißzange (225°C) zugeschweißt. Die Testzelle wird mit dem Vakuumschweißgerät evakuiert und verschlossen.
- Spannung und Widerstand der Testzelle werden mit einem handelsüblichen Multimeter überprüft und zwischen -0,200 bis 0,200 V bzw. zwischen 0,05 und 1 Ohm liegen. Zellen, die außerhalb der Werte liegen, werden nicht verwendet.

### Messprogramm

- Alle Testzellen werden zwischen 3,0V und 4,2V bei 25°C gezykelt. Hierbei werden folgende Stromraten verwendet:
   ∘ Phase 1:
- Zyklus 1 -50: Laderate:0,3C Entladerate 0,3C
   ∘ Phase 2:
- Zyklus 51: Laderate: 1C Entladerate 1C
- Zyklus 52: Laderate: 1C Entladerate 2C
- Zyklus 53: Laderate: 1 CEntladerate 4C
- Zyklus 54: Laderate: 1 CEntladerate 6C
- Zyklus 55: Laderate: 1 CEntladerate 8C
- Zyklus 56: Laderate: 1C Entladerate 10C
   ∘ Phase 3:
- Zyklus 57 - 107: Laderate: 1C Entladerate 1 C
   ∘ Weitere Phasen: Analog Phase 2 und 3
- Folgende Parameter werden pro Zyklus aufgenommen:
   ∘ Ladekapazität und Entladekapazität in mAh
   ∘ Innenwiderstand im geladenen Zustand (4,2V)
   ∘ Innenwiderstand im ungeladenen Zustand (3V)

Als Messgerät/Zyklisierer wird ein handelsübliches Gerät der Firma BaSyTec verwender Beispiel A: Referenz/Vergleichssystem
Der obengenannte Testsetup wird mit dem Elektrolytsystem 1M LiPF₆ in EC:DMC (1:1) durchgeführt (EC = Ethylencarbonat, DMC - Dimethylcarbonat)
Die Belastbarkeit (1 C -10C - Die Entladekapazität bei 1C wird als 100% gesetzt) zeigt Figur 1.

Die folgenden Tabellen geben die Ergebnisse wieder:

**Tabelle 1 gibt die Ergebnisse des Belastungstests wieder:**

| C-Rate | 1C | 2C | 4C | 6C | 8C | 10C |
|---|---|---|---|---|---|---|
| Relative Entladekapazität | 100% | 96% | 85% | 64% | 48% | 35% |

Figur 2 zeigt den Verlauf des Innenwiderstands im ungeladenen Zustand (3,0V).

**Tabelle 2 ist eine Zusammenfassung des Zyklentests (die Entladekapazität im 10. Zyklus wird auf 100% gesetzt)**

| | | | | | | | |
|---|---|---|---|---|---|---|---|
| Zyklus | 50 | 100 | 200 | 300 | 400 | 500 | |
| Rel. Entladekapazität | 99 | 94 | 93 | 93 | 92 | 91 | % |
| Innenwiderstand bei 3,0V | 0,9 | 1,0 | 1,1 | 1,3 | 1,4 | 1,6 | Ohm |
| Innenwiderstand bei 4,2V | 3,7 | 3,6 | 3,8 | 4,2 | 4,7 | 4,9 | Ohm |

**Tabelle 3 ist eine Zusammenfassung der relativen Entladekapazität bei 10C (die Entladekapazität der 1. 1C-Entladung wird auf 100% gesetzt)**

| | | | | | | | | |
|---|---|---|---|---|---|---|---|---|
| Nummer der Wiederholung des Belastungstests | 1 | 2 | 3 | 4 | 5 | 6 | 7 | |
| Relative Entladekapazität bei 10C | 35 | 30 | 30 | 25 | 25 | 23 | 20 | % |

Zusammenfassend zeigt sich bei der ersten Belastung mit 10C eine entnehmbare Kapazität von knapp 35% (Figur 1). Diese fällt mit zunehmender Wiederholung der Belastung. Beim 7. Durchlauf (ca. 500. Zyklus Figur 2) wird lediglich eine entnehmbare Kapazität von ca. 20% gemessen. Selbst nach der ersten starken Belastung von 10C erreicht die Zelle nicht die zuvor gemessenen Kapazitätswerte (Figur 2). Zugleich ist auffällig, dass der Innenwiderstand im geladenen Zustand über die Zyklenzahl (Figur 3) leicht, im ungeladenen Zustand (Figur 4) stark ansteigt.

Beispiel B: Elektrolyt, enthaltend 1-(3-(Methoxysulfonyl)propyl)-1- methylpyrrolidinium-tris(pentafluorethyl)trifluorophosphat

Der obengenannte Testsetup wird mit dem Elektrolytsystem 1M LiPF₆ in EC:DMC (1:1) + 1% 1-(3-(methoxysulfonyl)propyl)-1-methylpyrrolidinium-tris(pentafluorethyl)trifluorophosphat durchgeführt.

Die Figuren 3 und 4 sowie die Tabellen 4 bis 6 zeigen die Ergebnisse. Figur 3 zeigt die Belastbarkeit 1C bis 10C: die Entladekapazität bei 1C wird als 100% gesetzt.

**Die Tabelle 4 zeigt die Ergebnisse des Belastungstests:**

| | | | | | | |
|---|---|---|---|---|---|---|
| C-Rate | 1C | 2C | 4C | 6C | 8C | 10C |
| Relative Entladekapazität | 100% | 97% | 89% | 70% | 48% | 32% |

Den Verlauf des Innenwiderstands im ungeladenen Zustand (3V) zeigt Figur 4.

**Die Tabelle 5 gibt eine Zusammenfassung des Zyklentests (die Entladekapazität im 10. Zyklus wird auf 100% gesetzt).**

| | | | | | | |
|---|---|---|---|---|---|---|
| Zyklus | 50 | 100 | 200 | 300 | 400 | |
| Rel. Entladekapazität | 100 | 98 | 98 | 99 | 98 | % |
| Innenwiderstand bei 3V | 0,94 | 0,95 | 0,99 | 1,02 | 1,05 | Ohm |
| Innenwiderstand bei 4,2V | 2,07 | 2,14 | 1,99 | 2,02 | 2,14 | Ohm |
| | | | | | | |

**Die Tabelle 6 gibt eine Zusammenfassung der relativen Entladekapazität bei 10C (die Entladekapazität der 1. 1C-Entladung wird auf 100% gesetzt).**

| | | | | | | | | |
|---|---|---|---|---|---|---|---|---|
| Nummer der Wiederholung des Belastungstests | 1 | 2 | 3 | 4 | 5 | 6 | 7 | |
| Relative Entladekapazität bei 10C | 32 | 25 | 28 | 25 | 27 | 26 | 25 | % |

Zusammenfassend zeigt sich bei der ersten Belastung mit 10C eine entnehmbare Kapazität von 32% (Figur 5). Anders als bei der Referenz fällt mit zunehmender Wiederholung der Belastung signifikant weniger ab und erreicht auch nach dem siebten Durchlauf (400 Zyklen) noch 25%. Die Referenz fällt unter gleichen Bedingungen auf 20%
Anders als in der Referenzzelle (Beispiel A) wird selbst nach mehrfacher Belastung mit 10C die in den ersten Zyklen gemessene Kapazität wieder erhalten. Die Zelle altert deutlich langsamer und zeigt ein erstaunlich geringes Kapazitätsfading.

Zugleich ist der sehr konstante Verlauf der Innenwiderstände auffällig. Insbesondere im entladenen Zustand bleibt er über 400 Zyklen sehr konstant und zeigt im Verglich zur Referenz eine Verringerung um den Faktor 2.

Beispiel C: Elektrolyt enthaltend 1-Methyl-1-(2-(methylsulfoxy)ethyl)-pyrrolidinium-tris(pentafluorethyl)trifluorophosphat

Der obengenannte Testsetup wird mit dem Elektrolytsystem 1 M LiPF₆ in EC:DMC (1:1) + 1% 1-Methyl-1-(2-(methylsulfoxy)ethyl)- pyrrolidinium- tris(pentafluorethyl)trifluorophosphat durchgeführt.
Die Figuren 5 und 6 sowie die Tabellen 7 bis 9 zeigen die Ergebnisse

Figur 5 zeigt die Belastbarkeit 1C bis 10C: die Entladekapazität bei 1C wird als 100% gesetzt.

**Tabelle 7 zeigt die Ergebnisse des Belastungstests:**

| | | | | | | |
|---|---|---|---|---|---|---|
| C-Rate | 1C | 2C | 4C | 6C | 8C | 10C |
| Relative Entladekapazität | 100% | 98% | 90% | 71% | 54% | 43% |

Figur 6 zeigt den Verlauf des Innenwiderstands im ungeladenen Zustand (3,0V).

**Die Tabelle 8 zeigt die Zusammenfassung des Zyklentests (die Entladekapazität im 10. Zyklus wird auf 100% gesetzt).**

| | | | | | | |
|---|---|---|---|---|---|---|
| Zyklus | 50 | 100 | 200 | 300 | 400 | |
| Rel. Entladekapazität | 100 | 98 | 96 | 95 | 95 | % |
| Innenwiderstand bei 3,0V | 0,9 | 0,9 | 1,0 | 1,0 | 1,1 | Ohm |
| Innenwiderstand bei 4,2V | 3,6 | 2,1 | 2,2 | 2,3 | 2,3 | Ohm |

**Die Tabelle 9 gibt eine Zusammenfassung der relativen Entladekapazität bei 10C (die Entladekapazität der 1. 1C-Entladung wird auf 100% gesetzt).**

| | | | | | | | | |
|---|---|---|---|---|---|---|---|---|
| Nummer der Wiederholung des Belastungstests | 1 | 2 | 3 | 4 | 5 | 6 | | |
| Relative Entladekapazität | 43 | 42 | 39 | 39 | 35 | 36 | | % |
| bei 10C | | | | | | | | |

Zusammenfassend zeigt sich bei der ersten Belastung mit 10C eine im Vergleich zur Referenz deutlich höhere entnehmbare Kapazität von 43% (Figur 5).
Zugleich ist wie bei Beispiel B der sehr konstante Verlauf der Innenwiderstände auffällig. Dieser liegt insbesondere im entladenen Zustand, wie bei Beispiel B, bei sehr niedrigen Werten und deutlich unter der Referenz (Verringerung um den Faktor 2.)
Auch mit diesem Additiv wird eine deutlich geringere Alterung und Kapazitätsfading erreicht.

Beispiel D: Elektrolyt enthaltend 1-Methyl-1-(2-(methylsulfoxy)ethyl)- pyrrolidinium-bis(oxalato)borat

Der obengenannte Testsetup wird mit dem Elektrolytsystem 1 M LiPF₆ in EC:DMC (1:1) + 1% 1-Methyl-1-(2-(methylsulfoxy)ethyl)- pyrrolidinium- bis(oxalato)borat durchgeführt.

Die Figuren 7 und 8 sowie die Tabellen 10 bis 12 zeigen die Ergebnisse.

Figur 7 zeigt die Belastbarkeit 1C bis 10C: Die Entladekapazität bei 1 C wird als 100% gesetzt.

**Tabelle 10 zeigt den Belastungstest:**

| | | | | | | |
|---|---|---|---|---|---|---|
| C-Rate | 1C | 2C | 4C | 6C | 8C | 10C |
| Relative Entladekapazität | 100% | 98% | 85% | 68% | 52% | 40% |

Figur 8 zeigt den Verlauf des Innenwiderstands im ungeladenen Zustand (3,0V).

**Tabelle 11 zeigt die Zusammenfassung des Zyklentests (die Entladekapazität im 10. Zyklus wird auf 100% gesetzt).**

| | | | | | | |
|---|---|---|---|---|---|---|
| Zyklus | 50 | 100 | 200 | 300 | 400 | |
| Rel. Entladekapazität | 98 | 97 | 96 | 96 | | % |
| Innenwiderstand bei 3,0V | 0,99 | 0,98 | 0,99 | 0,99 | | Ohm |
| Innenwiderstand bei 4,2V | 2,2 | 2,1 | 2,0 | 2,0 | | Ohm |

**Tabelle 12 zeigt die Zusammenfassung der relativen Entladekapazität bei 10C (die Entladekapazität der 1. 1 C Entladung wird auf 100% gesetzt).**

| | | | | | | | | |
|---|---|---|---|---|---|---|---|---|
| Nummer der Wiederholung des Belastungstests | 1 | 2 | 3 | 4 | | | | |
| Relative Entladekapazität bei 10C | 40 | 39 | 39 | 38 | | | | % |

Ähnlich wie in den Beispielen B und C zeigt sich bei der Belastung mit 10C eine im Vergleich zur Referenz deutlich höhere entnehmbare Kapazität von 40% Zugleich ist wie bei Beispiel B und C der sehr konstante Verlauf der Innenwiderstände auffällig. Dieser liegt insbesondere im entladenen Zustand deutlich unter der Referenz.
Verzeichnis der Figuren:
Figur 1 zeigt die Belastbarkeit 1C bis 10C des Referenzsystems 1M LiPF₆ in EC:DMC (1:1).
Figur 2 zeigt den Verlauf des Innenwiderstands im ungeladenen Zustand (3,0V) des Referenzsystems 1M LiPF₆ in EC:DMC (1:1).
Figur 3 zeigt die Belastbarkeit 1 C bis 10C des Beispiels B 1 M LiPF₆ in EC:DMC (1:1), 1% 1-(3-(Methoxysulfonyl)propyl)-1-methylpyrrolidinium-tris(pentafluorethyl)trifluorophosphat
Figur 4 zeigt den Verlauf des Innenwiderstands im ungeladenen Zustand (3,0V) des Beispiels B 1M LiPF₆ in EC:DMC (1:1), 1% 1-(3-(Methoxysulfonyl)propyl)-1-methylpyrrolidinium-tris(pentafluorethyl)trifluorophosphat.
Figur 5 zeigt die Belastbarkeit 1C bis 10C des Beispiels C 1M LiPF₆ in EC:DMC (1:1), 1% 1-Methyl-1-(2-(methylsulfoxy)ethyl)-pyrrolidinium-tris(pentafluorethyl)trifluorophosphat.
Figur 6 zeigt den Verlauf des Innenwiderstands im ungeladenen Zustand (3,0V) des Beispiels C 1 M LiPF₆ in EC:DMC (1:1), 1% 1-Methyl-1-(2-(methylsulfoxy)ethyl)-pyrrolidinium-tris(pentafluorethyl)trifluorophosphat.
Figur 7 zeigt die Belastbarkeit 1C bis 10C des Beispiels D 1M LiPF₆ in EC:DMC (1:1), 1% 1-Methyl-1-(2-(methylsulfoxy)ethyl)-pyrrolidinium-bis(oxalato)borat.
Figur 8 zeigt den Verlauf des Innenwiderstands im ungeladenen Zustand (3,0V) des Beispiels D 1 M LiPF₆ in EC:DMC (1:1), 1% 1-Methyl-1-(2-(methylsulfoxy)ethyl)-pyrrolidinium-bis(oxalato)borat.

## Patentansprüche

1. Verbindungen der Formel I
[K-(CH₂)-(CH₂)ᵤ-SO₃-(CH₂)ᵥ-R"]⁺ [A]- I,
wobei
u 0, 1, 2, 3, 4, 5, 6 oder 7 bedeutet und wobei mindestens eine CH₂-Gruppe der-(CH₂)ᵤ-Alkylenkette mit u = 2, 3, 4, 5, 6 oder 7 durch O ersetzt sein kann oder mindestens eine Doppelbindung enthalten kann,
v 0, 1, 2, 3 oder 4 bedeutet, 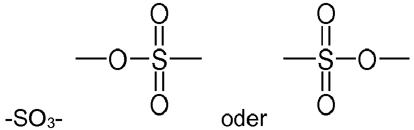 bedeutet,
R" jeweils unabhängig voneinander eine geradkettige oder verzweigte Alkylgruppe mit 1 bis 8 C-Atomen, die nicht fluoriert, teilweise fluoriert oder vollständig fluoriert sein kann, eine geradkettige oder verzweigte Alkenylgruppe mit 2 bis 20 C-Atomen und einer oder mehrerer Doppelbindungen, eine geradkettige oder verzweigte Alkinylgruppe mit 2 bis 20 C-Atomen und einer oder mehrerer Dreifachbindungen oder eine Arylgruppe mit 6 bis 12 C-Atomen, die durch F, Cl und/oder einer geradkettige oder verzweigte, teilweise fluorierte oder vollständig fluorierte Alkylgruppe mit 1 bis 8 C-Atomen einfach oder mehrfach subsituiert sein kann, bedeutet,
K ein Kation ist, ausgewählt aus der Gruppe
R₃N⁺-*, R₃P⁺-*, 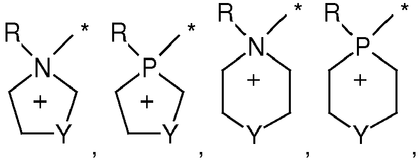 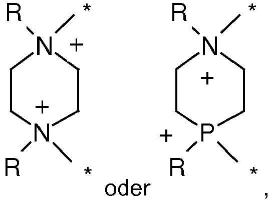 wobei
R jeweils unabhängig voneinander Methyl, Ethyl, n-Propyl, iso-Propyl, n-Butyl, sec.-Butyl oder tert.-Butyl bedeutet, wobei mindestens eine, nicht direkt mit N oder P verbundenen, CH₂-Gruppe der genannten Reste R durch O ersetzt sein kann,
Y CH₂, O, S oder NR' bedeutet und
R' Methyl, Ethyl, n-Propyl, iso-Propyl, n-Butyl, sec.-Butyl oder tert.-Butyl bedeutet,
A ein Anion ist, ausgewählt aus der Gruppe
[F_{z}B(CₘF_{2m,1})_{4-z}]⁻,
[F_{y}P(CₘF₂ₘ₊₁)_{6-y}]⁻,
[(CₘF₂ₘ₊₁)₂P(O)O]⁻,
[CₘF₂ₘ₊₁P(O)O₂]²⁻,
[O-C(O)-CₘF₂ₘ₊₁]⁻,
[N(C(O)-CₘF₂ₘ₊₁)₂]⁻,
[N(S(O)₂-CₘF₂ₘ₊₁)₂]⁻,
[N(C(O)-CₘF₂ₘ₊₁)(S(O)₂-CₘF₂ₘ₊₁)]⁻,
[N(C(O)-CₘF₂ₘ₊₁)(C(O)F)]⁻,
[N(S(O)₂-CₘF₂ₘ₊₁)(S(O)₂F)]⁻,
[N(S(O)₂F)₂]⁻,
[C(C(O)-CₘF₂ₘ₊₁)₃]⁻,
[C(S(O)₂-CₘF₂ₘ₊₁)₃]⁻, 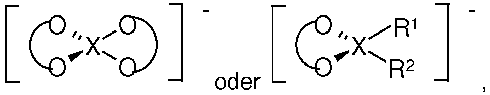 wobei
m 1, 2, 3, 4, 5, 6, 7 oder 8 bedeutet, wobei teilweise CF₂-Gruppen in den genannten Anionen durch O, S(O)₂, NR oder CH₂ ersetzt sein können,
z 1, 2 oder 3 bedeutet,
y 1, 2, 3, 4, 5 oder 6 bedeutet,
X B oder Al bedeutet,
R¹ und R² jeweils unabhängig voneinander F, Cl, Br, I, eine geradkettige oder verzweigte Perfluoralkylgruppe mit 1 bis 20 C-Atomen, eine geradkettige oder verzweigte Alkoxygruppe mit 1 bis 20 C-Atomen, die nicht fluoriert, teilweise fluoriert oder vollständig fluoriert sein kann, oder -O-C(O)-Alkyl bedeutet, wobei Alkyl eine geradkettige oder verzweigte Alkylgruppe mit 1 bis 20 C-Atomen bedeutet, die nicht fluoriert, teilweise fluoriert oder perfluoriert sein kann,
und
wobei 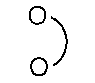 jeweils unabhängig voneinander ein zweizähniger Rest bedeutet, der sich von einem 1,2- oder 1,3- Diol, von einer 1,2 -oder 1,3- Dicarbonsäure oder einer 1,2- oder 1,3- Hydroxycarbonsäure ableitet, in dem jeweils zwei benachbarte OH-Gruppen der entsprechenden Verbindung mit dem Zentralatom X jeweils eine Bindung eingehen und dabei zwei H-Atome formal abgespaltet werden,
und wobei die Elektroneutralität des entsprechenden Salzes der Formel I zu beachten ist.

2. Verbindungen nach Anspruch 1, **dadurch gekennzeichnet, dass** v 0 bedeutet.

3. Verbindungen nach Anspruch 1 oder 2, **dadurch gekennzeichnet, dass** u 1, 2 oder 3 bedeutet.

4. Verbindungen nach einem oder mehreren der Ansprüche 1 bis 3, **dadurch gekennzeichnet, dass** R" eine geradkettige oder verzweigte Alkylgruppe mit 1 bis 8 C-Atomen bedeutet, die nicht fluoriert, teilweise fluoriert oder vollständig fluoriert sein kann.

5. Verbindungen nach einem oder mehreren der Ansprüche 1 bis 4, **dadurch gekennzeichnet, dass** K dem Kation 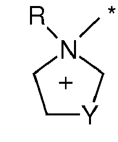 entspricht, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4.

6. Verbindungen nach einem oder mehreren der Ansprüche 1 bis 5, **dadurch gekennzeichnet, dass** A ein Anion ist, ausgewählt aus der Gruppe
[F_{z}B(CₘF_{2m,1})_{4-z}]⁻,
[F_{y}P(CₘF₂ₘ₊₁)_{6-y}]⁻,
[O-C(O)-CₘF₂ₘ₊₁]⁻,
[N(S(O)₂-CₘF₂ₘ₊₁)₂]⁻,
[N(S(O)₂F)₂]⁻, 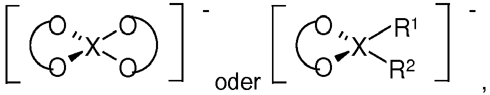 wobei
m 1, 2, 3, 4, 5, 6, 7 oder 8 bedeutet,
z 1, 2 oder 3 bedeutet,
y 3, 4, 5 oder 6 bedeutet,
X B bedeutet,
R¹ und R² jeweils unabhängig voneinander F, eine geradkettige oder verzweigte Perfluoralkylgruppe mit 1 bis 4 C-Atomen, eine geradkettige oder verzweigte Alkoxygruppe mit 1 bis 4 C-Atomen oder -O-C(O)-Alkyl bedeutet, wobei Alkyl eine geradkettige oder verzweigte Alkylgruppe mit 1 bis 20 C-Atomen bedeutet, die nicht fluoriert, teilweise fluoriert oder perfluoriert sein kann,
und
wobei 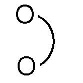 jeweils unabhängig voneinander ein zweizähniger Rest bedeutet, der sich von einem 1,2- oder 1,3- Diol, von einer 1,2 -oder 1,3- Dicarbonsäure oder einer 1,2- oder 1,3- Hydroxycarbonsäure ableitet, in dem jeweils zwei benachbarte OH-Gruppen der entsprechenden Verbindung mit dem Zentralatom X jeweils eine Bindung eingehen und dabei zwei H-Atome formal abgespaltet werden.

7. Verfahren zur Herstellung von Verbindungen der Formel I nach einem oder mehreren der Ansprüche 1 bis 6, wobei SO₃ der Teilstruktur 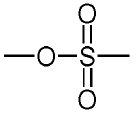 entspricht, **dadurch gekennzeichnet, dass** eine Verbindung der Formel (II)
K-1 (II),
wobei K-1 ausgewählt wird aus der Gruppe R₃N, R₃P, 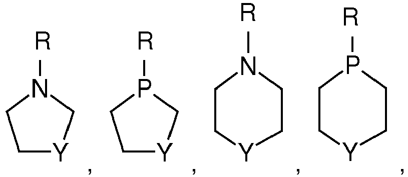 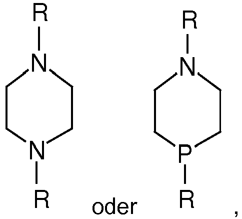 wobei
R jeweils unabhängig voneinander Methyl, Ethyl, n-Propyl, iso-Propyl, n-Butyl, sec.-Butyl oder tert.-Butyl bedeutet, wobei mindestens eine, nicht direkt mit N oder P verbundenen, CH₂-Gruppe der genannten Reste R durch O ersetzt sein kann,
Y CH₂, O, S oder NR' bedeutet und
R' Methyl, Ethyl, n-Propyl, Isopropyl, n-Butyl, sec.-Butyl oder tert.-Butyl bedeutet, zunächst mit einer Verbindung der Formel (III) umgesetzt wird,
Hal-(CH₂)-(CH₂)ᵤ-OH (III),
wobei u 1, 2, 3, 4, 5, 6 oder 7 bedeutet, wobei mindestens eine nicht an den Sauerstoff angebundene CH₂-Gruppe der -(CH₂)ᵤ-Alkylenkette mit u = 2, 3, 4, 5, 6 oder 7 durch O ersetzt sein kann oder mindestens eine Doppelbindung enthalten kann, und Hal Cl, Br oder I bedeutet,
und die entstehende Zwischenverbindung der Formel (IV)
[K-(CH₂)-(CH₂)ᵤ-OH]⁺ [Hal]- (IV),
wobei K, u und Hal eine der zuvor genannten Bedeutungen haben,
mit einer Verbindung der Formel (V)
R"-(CH₂)ᵥ-SO₂-L (V),
wobei v und R" eine Bedeutung nach Anspruch 1 haben und L eine Abgangsgruppe ausgewählt aus einer geradkettigen oder verzweigten Alkoxygruppe mit 1 bis 3 C-Atomen, Cl oder F bedeutet,
unter Säurekatalyse im Fall der Alkoxygruppe als Abgangsgruppe oder in Gegenwart einer Base im Fall von Cl oder F als Abgangsgruppe,
umgesetzt wird
und die entstehende Verbindung der Formel (VI)
[K-(CH₂)-(CH₂)ᵤ-SO₃-(CH₂)ᵥ-R"]⁺ [Hal]⁻ (VI),
in der K, u, v, R" und Hal eine der zuvor genannten Bedeutungen haben,
in einer Metathesereaktion mit einer Verbindung der Formel (VII) umgesetzt wird,
[Kt]⁺[A]⁻ (VII),
wobei [Kt]⁺ ein Alkalimetallkation oder H⁺ bedeutet und [A]- eine in Anspruch 1 angegebene Bedeutung hat.

8. Verfahren zur Herstellung von Verbindungen der Formel I nach einem oder mehreren der Ansprüche 1 bis 6, wobei SO₃ der Teilstruktur 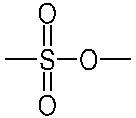 entspricht und u 2, 3, 4 oder 5 bedeutet, **dadurch gekennzeichnet, dass** eine Verbindung der Formel (II)
K-1 (II),
wobei K-1 ausgewählt wird aus der Gruppe R₃N, R₃P, 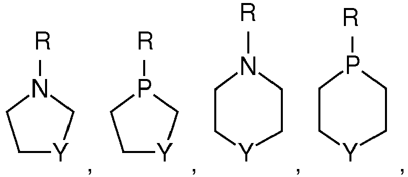 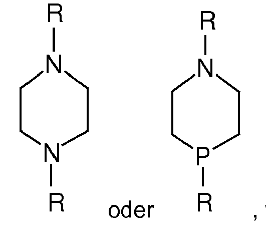 wobei
R jeweils unabhängig voneinander Methyl, Ethyl, n-Propyl, iso-Propyl, n-Butyl, sec.-Butyl oder tert.-Butyl bedeutet, wobei mindestens eine, nicht direkt mit N oder P verbundene CH₂-Gruppe der genannten Reste R durch O ersetzt sein kann,
Y CH₂, O, S oder NR' bedeutet und
R' Methyl, Ethyl, n-Propyl, iso-Propyl, n-Butyl, sec.-Butyl oder tert.-Butyl bedeutet, zunächst mit einer Verbindung der Formel (VIII) 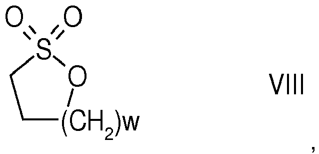 wobei w 1, 2, 3 oder 4 bedeutet,
zu der Zwischenverbindung der Formel (IX)
[K-(CH₂)-(CH₂)ᵤ-SO₃⁻] (IX),
wobei K und u eine in Anspruch 1 genannte Bedeutung haben,
umgesetzt wird,
und anschließend die Zwischenverbindung der Formel (IX), mit einem Alkylierungsmittel der Formel (X) umgesetzt wird
L*-(CH₂)ᵥ-R" (X),
wobei v und R" eine in Anspruch 1 angegebene Bedeutung haben und L* CF₃-S(O)₂O, C₄F₉-S(O)₂O, (C₂F₅)₂P(O)O, (C₄F₉)₂P(O)O, (Alkyl)₂O⁺, Alkyl-S(O)₂O, Alkyl-O-S(O)₂O, I oder Br bedeutet, wobei Alkyl jeweils unabhängig voneinander eine geradkettige oder verzweigte Alkylgruppe mit 1 bis 4 C-Atomen bedeutet,
und die erhaltene Verbindung der Formel (XI)
[K-(CH₂)-(CH₂)ᵤ-SO₃-(CH₂)ᵥ-R"]⁺ [L*]⁻ (XI),
wobei K, u, v, R" und L* eine zuvor genannte Bedeutung haben,
gegebenenfalls mit einer Verbindung der Formel (VII) umgesetzt wird,
[Kt]⁺[A]⁻ (VII),
wobei [Kt]⁺ ein Alkalimetallkation oder H⁺ bedeutet und [A]- eine in Anspruch 1 genannte Bedeutung hat, wenn das Anion [L*]⁻ nicht schon einer Bedeutung des Anions [A]- entspricht.

9. Verfahren zur Herstellung von Verbindungen der Formel I nach einem oder mehreren der Ansprüche 1 bis 6, wobei SO₃ der Teilstruktur 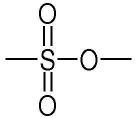 entspricht, **dadurch gekennzeichnet, dass** eine Verbindung der Formel (II) K-1 (II), wobei K-1 ausgewählt wird aus der Gruppe R₃N, R₃P, 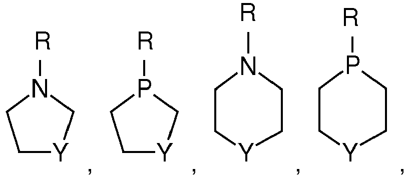 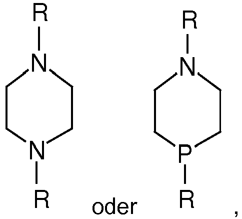 wobei
R jeweils unabhängig voneinander Methyl, Ethyl, n-Propyl, iso-Propyl, n-Butyl, sec.-Butyl oder tert.-Butyl bedeutet, wobei mindestens eine, nicht direkt mit N oder P verbundenen, CH₂-Gruppe der genannten Reste R durch O ersetzt sein kann,
Y CH₂, O, S oder NR' bedeutet und
R' Methyl, Ethyl, n-Propyl, iso-Propyl, n-Butyl, sec.-Butyl oder tert.-Butyl bedeutet, zunächst mit einer Verbindung der Formel (XII) umgesetzt wird,
Hal-(CH₂)-(CH₂)ᵤ-Hal (XII),
wobei u 0, 1, 2, 3, 4, 5, 6 oder 7 bedeutet, wobei mindestens eine nicht an Hal angebundene CH₂-Gruppe der -(CH₂)ᵤ-Alkylenkette mit u = 2, 3, 4, 5, 6 oder 7 durch O ersetzt sein kann oder mindestens eine Doppelbindung enthalten kann, und Hal Cl, Br oder I bedeutet, und die entstehende Zwischenverbindung der Formel (XIII)
[K-(CH₂)-(CH₂)ᵤ-Hal]⁺ [Hal]- (XIII),
wobei K, u und Hal eine der zuvor genannten Bedeutungen haben,
mit einem Alkalimetallsulfit oder Ammoniumsulfit und anschließend mit einer Verbindung der Formel (X)
L*-(CH₂)ᵥ-R" (X),
wobei v und R" eine in Anspruch 1 angegebene Bedeutung haben und L* CF₃-S(O)₂O, C₄F₉-S(O)₂O, (C₂F₅)₂P(O)O, (C₄F₉)₂P(O)O, (Alkyl)₂O⁺, Alkyl-S(O)₂O, Alkyl-O-S(O)₂O, I oder Br bedeutet, wobei Alkyl jeweils unabhängig voneinander eine geradkettige oder verzweigte Alkylgruppe mit 1 bis 4 C-Atomen bedeutet,
umgesetzt wird
und die entstehende Verbindung der Formel (XI)
[K-(CH₂)-(CH₂)ᵤ-SO₃-(CH₂)ᵥ-R"]⁺ [L*]⁻ (XI),
in der K, u, v, R" und L* eine der zuvor genannten Bedeutungen haben,
gegebenenfalls mit einer Verbindung der Formel (VII) umgesetzt wird,
[Kt]⁺[A]⁻ (VII),
wobei [Kt]⁺ ein Alkalimetallkation oder H⁺ bedeutet und [A]- eine in Anspruch 1 genannte Bedeutung hat, wenn das Anion [L*]⁻ nicht schon einer Bedeutung des Anions [A]- entspricht.

10. Elektrolyt, enthaltend mindestens eine Verbindung der Formel I nach einem oder mehreren der Ansprüche 1 bis 6, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4.

11. Elektrolyt nach Anspruch 10, **dadurch gekennzeichnet, dass** als Leitsalz ein Lithiumsalz und/oder ein Tetraalkylammoniumsalz enthalten ist.

12. Elektrolyt nach Anspruch 10 oder 11, **dadurch gekennzeichnet, dass** ein oder mehrereweitere Additive enthalten sind.

13. Elektrochemische oder elektrooptische Vorrichtung, enthaltend mindestens eine Verbindung der Formel I nach einem oder mehreren der Ansprüche 1 bis 6, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4.

14. Verwendung von Verbindungen der Formel I nach einem oder mehreren der Ansprüche 1 bis 6, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, als Additiv in Elektrolyten.

15. Verwendung von Verbindungen der Formel I nach einem oder mehreren der Ansprüche 1 bis 6, wobei [A]- auch ausgewählt sein kann aus [O-S(O)₂-CₘF₂ₘ₊₁]⁻ mit m = 1, 2, 3, 4, 5, 6, 7 oder 8 und [F_{z}B(CₘF₂ₘ₊₁)_{4-z}]⁻ mit z = 4, in elektrochemischen oder elektrooptischen Vorrichtungen.

16. Verwendung nach Anspruch 15, **dadurch gekennzeichnet, dass** die elektrochemische oder elektrooptische Vorrichtung eine Lithium-Batterie, eine Lithium-Ionen-Batterie, ein Doppelschichtkondensator, ein Lithium-Kondensator, eine Solarzelle, ein elektrochromes Display, ein Sensor oder ein Biosensor ist.

## Claims

1. A compound of the formula I
[K-(CH₂)-(CH₂)ᵤ-SO₃-(CH₂)ᵥ-R"]⁺ [A]⁻ I,
where
u is 0, 1, 2, 3, 4, 5, 6 or 7 and where at least one CH₂ group of the -(CH₂)ᵤ-alkylene chain with u = 2, 3, 4, 5, 6 or 7 may be replaced by 0 or may contain at least one double bond,
v is 0, 1, 2, 3 or 4, -SO₃- denotes 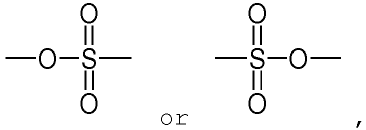
R" independently at each occurrence is a straight-chain or branched alkyl group having 1 to 8 C atoms, and may be unfluorinated, partly fluorinated or fully fluorinated, or is a straight-chain or branched alkenyl group having 2 to 20 C atoms and one or more double bonds, a straight-chain or branched alkynyl group having 2 to 20 C atoms and one or more triple bonds, or an aryl group having 6 to 12 C atoms, which group may be singly or multiply substituted by F, Cl and/or a straight-chain or branched, partly fluorinated or fully fluorinated alkyl group having 1 to 8 C atoms,
K is a cation selected from the group
R₃N⁺-*, R₃P⁺-*, 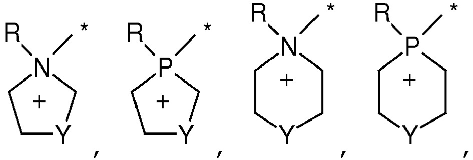 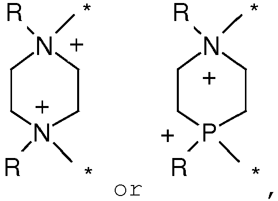 where
R independently at each occurrence is methyl, ethyl, n-propyl, isopropyl, n-butyl, sec-butyl or tert-butyl, it being possible for at least one CH₂ group, not directly joined to N or P, in the stated radicals R to be replaced by 0,
Y is CH₂, 0, S or NR', and
R' is methyl, ethyl, n-propyl, isopropyl, n-butyl, sec-butyl or tert-butyl,
A is an anion selected from the group
[F_{z}B (CₘF₂ₘ₊₁)_{4-z}]⁻,
[F_{y}P(CₘF₂ₘ₊₁)_{6-y}]⁻,
[(_{C}mF₂ₘ₊₁)₂P(O)O]⁻,
[CₘF₂ₘ₊₁P(O)O₂]2⁻,
[O-C(O)-CₘF₂ₘ₊₁]⁻,
[N(C(O)-CₘF₂ₘ₊₁)₂]⁻,
[N(S(O)₂-CₘF₂ₘ₊₁)₂]⁻,
[N(C(O)-CₘF₂ₘ₊₁)(S(O)₂-CₘF₂ₘ₊₁)]⁻,
[N(C(O)-CₘF₂ₘ₊₁)(C(O)F)]⁻,
[N(S(O)₂-CₘF₂ₘ₊₁) (S(O)₂F)]⁻,
[N(S(O)₂F)₂]⁻,
[C(C(O)-CₘF₂ₘ₊₁)₃]⁻,
[C(S(O)₂-CₘF₂ₘ₊₁)₃]⁻, where 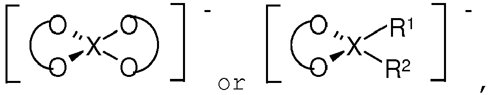
m is 1, 2, 3, 4, 5, 6, 7 or 8, it being possible partly for CF₂ groups in the stated anions to be replaced by O, S(O)₂, NR or CH₂,
z is 1, 2 or 3,
y is 1, 2, 3, 4, 5 or 6,
X is B or Al,
R¹ and R² in each case independently of one another are F, Cl, Br, I, a straight-chain or branched perfluoroalkyl group having 1 to 20 C atoms, a straight-chain or branched alkoxy group having 1 to 20 C atoms, which may be unfluorinated, partly fluorinated or fully fluorinated, or -O-C(O)-alkyl, where alkyl is a straight-chain or branched alkyl group having 1 to 20 C atoms, and may be unfluorinated, partly fluorinated or perfluorinated,
and
where 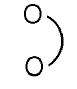 independently at each occurrence is a bidentate radical which derives from a 1,2- or 1,3-diol, from a 1,2- or 1,3-dicarboxylic acid or from a 1,2- or 1,3-hydroxycarboxylic acid, by the entering of pairs of adjacent OH groups in the compound in question into one bond each to the central atom X, accompanied by formal elimination of two H atoms, and with the electroneutrality of the corresponding salt of the formula I being observed.

2. A compound according to claim 1, wherein v is 0.

3. A compound according to claim 1 or 2, wherein u is 1, 2 or 3.

4. A compound according to one or more of claims 1 to 3, wherein R" is a straight-chain or branched alkyl group having 1 to 8 C atoms, and may be unfluorinated, partly fluorinated or fully fluorinated.

5. A compound according to one or more of claims 1 to 4, wherein K corresponds to the cation 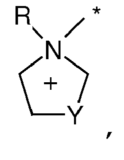 where [A]⁻ can also be selected from [O-S(O)₂-CₘF₂ₘ₊₁]⁻ with m = 1, 2, 3, 4, 5, 6, 7 or 8 and [F_{z}B(CₘF₂ₘ₊₁)_{4-z}]⁻ with z = 4.

6. A compound according to one or more of claims 1 to 5, wherein A is an anion selected from the group
[F_{z}B(CₘF₂ₘ₊₁)_{4-z}]⁻,
[F_{y}P(CₘF₂ₘ₊₁)_{6-y}]⁻,
[O-C(O)-CₘF₂ₘ₊₁]⁻,
[N(S(O)₂CₘF₂ₘ₊₁)₂]⁻,
[N(S(O)₂F)₂]⁻, 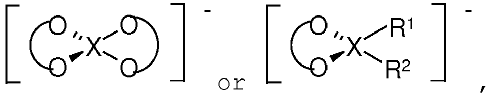 where
m is 1, 2, 3, 4, 5, 6, 7 or 8,
z is 1, 2 or 3,
y is 3, 4, 5 or 6,
X is B,
R¹ and R² in each case independently of one another are F, a straight-chain or branched perfluoroalkyl group having 1 to 4 C atoms, a straight-chain or branched alkoxy group having 1 to 4 C atoms or -0-C(O)-alkyl, where alkyl is a straight-chain or branched alkyl group having 1 to 20 C atoms, and may be unfluorinated, partly fluorinated or perfluorinated,
and
where 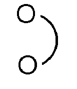 independently at each occurrence is a bidentate radical which derives from a 1,2- or 1,3-diol, from a 1,2- or 1,3-dicarboxylic acid or from a 1,2- or 1,3-hydroxycarboxylic acid, by the entering of pairs of adjacent OH groups in the compound in question into one bond each to the central atom X, accompanied by formal elimination of two H atoms.

7. A process for preparing a compound of the formula I according to one or more of claims 1 to 6, where SO₃ corresponds to the substructure 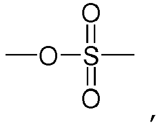 wherein a compound of the formula (II)
K-1 (II),
where K-1 is selected from the group R₃N, R₃P, 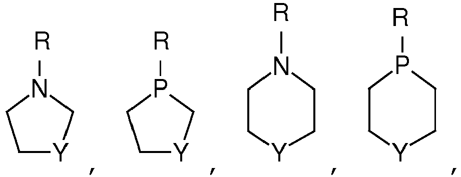 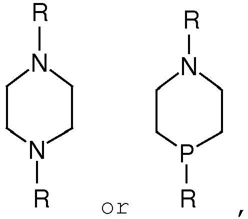 where
R independently at each occurrence is methyl, ethyl, n-propyl, isopropyl, n-butyl, sec-butyl or tert-butyl, and at least one CH₂ group, not joined to N or P, in the stated radicals R may be replaced by O,
Y is CH₂, O, S or NR', and
R' is methyl, ethyl, n-propyl, isopropyl, n-butyl, sec-butyl or tert-butyl,
is first reacted with a compound of the formula (III),
Hal-(CH₂)-(CH₂)ᵤ-OH (III),
where u is 1, 2, 3, 4, 5, 6 or 7, and at least one CH₂ group, not attached to the oxygen, in the-(CH₂)ᵤ-alkylene chain with u = 2, 3, 4, 5, 6 or 7 may be replaced by O or may contain at least one double bond, and Hal is Cl, Br or I,
and the resultant intermediate compound of the formula (IV)
[K-(CH₂)-(CH₂)ᵤ-OH]⁺ + [Hal]⁻ (IV),
where K, u and Hal have one of the above-stated definitions,
is reacted with a compound of the formula (V)
R''-(CH₂)ᵥ-SO₂-L (V),
where v and R" have a definition according to claim 1 and L is a leaving group selected from a straight-chain or branched alkoxy group having 1 to 3 C atoms, Cl or F,
with acid catalysis in the case of the alkoxy group as leaving group, or in the presence of a base in the case of Cl or F as leaving group,
and the resulting compound of the formula (VI)
[K-(CH₂)-(CH₂)ᵤ-SO₃(CH₂)ᵥ-R"]⁺ [Hal]⁻ (VI),
in which K, u, v, R" and Hal have one of the above-stated definitions,
is reacted in a metathesis reaction with a compound of the formula (VII),
[Kt]⁺[A]⁻ (VII),
where [Kt]⁺ is an alkali metal cation or H⁺, and [A]⁻ has a definition indicated in claim 1.

8. A process for preparing a compound of the formula I according to one or more of claims 1 to 6, where SO₃ corresponds to the substructure 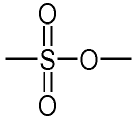 and u is 2, 3, 4 or 5, wherein a compound of the formula (II)
K-1 (II),
where K-1 is selected from the group R₃N, R₃P, 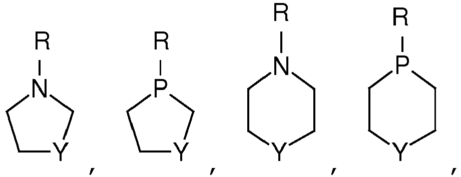 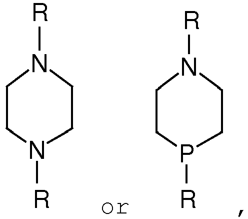 where
R independently at each occurrence is methyl, ethyl, n-propyl, isopropyl, n-butyl, sec-butyl or tert-butyl, and at least one CH₂ group, not joined to N or P, in the stated radicals R may be replaced by O,
Y is CH₂, O, S or NR', and
R' is methyl, ethyl, n-propyl, isopropyl, n-butyl, sec-butyl or tert-butyl,
is first reacted with a compound of the formula (VIII) 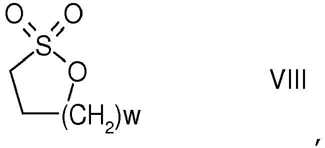 where w is 1, 2, 3 or 4,
to give the intermediate compound of formula (IX)
[K- (CH₂) - (CH₂)ᵤ-SO₃⁻] (IX),
where K and u have a definition stated in claim 1, and subsequently the intermediate compound of the formula (IX) is reacted with an alkylating agent of the formula (X)
L*-(CH₂)ᵥ-R" (X),
where v and R'' have a definition indicated in claim 1 and L* is CF₃-S(O)₂O, C₄F₉-S(O)₂O, (C₂F₅)₂P(O)O, (C₄F₉)₂P(O)O, (alkyl)₂O⁺, alkyl-S(O)₂O, alkyl-O-S(O)₂O, I or Br, with alkyl independently at each occurrence being a straight-chain or branched alkyl group having 1 to 4 C atoms,
and the resulting compound of the formula (XI)
[K-(CH₂)-(CH₂)ᵤ-SO₃(CH₂)ᵥ-R'']⁺[L*]⁻ (XI),
where K, u, v, R" and L* have an above-stated definition,
is reacted optionally with a compound of the formula (VII),
[Kt]⁺[A]⁻ (VII),
where [Kt]⁺ is an alkali metal cation or H⁺, and [A]⁻ has a definition stated in claim 1, if the anion [L*]⁻ does not already correspond to a definition of the anion [A]⁻.

9. A process for preparing a compound of the formula I according to one or more of claims 1 to 6, where SO₃ corresponds to the substructure 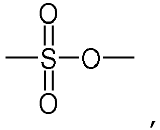 wherein a compound of the formula (II)
K-1 (II),
where K-1 is selected from the group R₃N, R₃P, 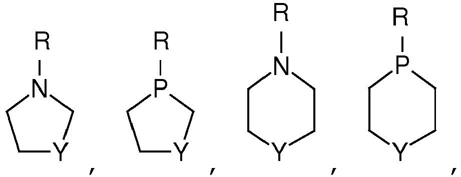 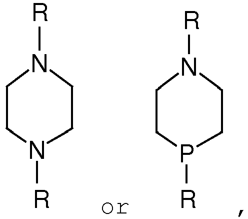 where
R independently at each occurrence is methyl, ethyl, n-propyl, isopropyl, n-butyl, sec-butyl or tert-butyl, and at least one CH₂ group, not joined to N or P, in the stated radicals R may be replaced by 0,
Y is CH₂, 0, S or NR', and
R' is methyl, ethyl, n-propyl, isopropyl, n-butyl, sec-butyl or tert-butyl, is first reacted with a compound of the formula (XII),
Hal-(CH₂)-(CH₂)ᵤ-Hal (XII),
where u is 0, 1, 2, 3, 4, 5, 6 or 7, and at least one CH₂ group, not attached to Hal, in the -(CH₂)ᵤ-alkylene chain with u = 2, 3, 4, 5, 6 or 7 may be replaced by 0 or may contain at least one double bond, and Hal is Cl, Br or I,
and the resultant intermediate compound of the formula (XIII)
[K-(CH₂)-(CH₂)ᵤ-Hal]⁺[Hal]⁻ (XIII),
where K, u and Hal have one of the above-stated definitions,
is reacted with an alkali metal sulfite or ammonium sulfite and subsequently with a compound of the formula (X)
L*-(CH₂)ᵥ-R" (X),
where v and R" have a definition indicated in claim 1 and L* is CF₃-S(O)₂O, C₄F₉-S(O)₂O, (C₂F₅)₂P(O)O, (C₄F₉)₂P(O)O, (alkyl)₂O⁺, alkyl-S(O)₂O, alkyl-O-S(O)₂O, I or Br, with alkyl independently at each occurrence being a straight-chain or branched alkyl group having 1 to 4 C atoms,
and the resulting compound of the formula (XI)
[K-(CH₂)-(CH₂)ᵤ-SO₃(CH₂)ᵥ-R"]⁺[L*]⁻ (XI),
where K, u, v, R" and L* have one of the above-stated definitions,
is reacted optionally with a compound of the formula (VII),
[Ktl⁺[A]⁻ (VII),
where [Kt]⁺ is an alkali metal cation or H⁺, and [A]⁻ has a definition stated in claim 1, if the anion [L*]⁻ does not already correspond to a definition of the anion [A]⁻.

10. An electrolyte comprising at least one compound of the formula I according to one or more of claims 1 to 6 where [A]⁻ can also be selected from [O-S)O)₂⁻ CₘF₂ₘ₊₁]⁻ with m = 1, 2, 3, 4, 5, 6, 7 or 8 and [F_{z}B(CₘF₂ₘ₊₁)_{4-z}]⁻ with z = 4.

11. The electrolyte according to claim 10, comprising as conductive salt a lithium salt and/or a tetraalkylammonium salt.

12. The electrolyte according to claim 10 or 11, comprising one or more further additives.

13. An electrochemical or electrooptical device, comprising at least one compound of the formula I according to one or more of claims 1 to 6, where [A]⁻ can also be selected from [O-S(O)₂-CₘF₂ₘ₊₁]⁻ with m = 1, 2, 3, 4, 5, 6, 7 or 8 and [F_{z}B(CₘF₂ₘ₊₁)_{4-z}]⁻ with z = 4.

14. The use of compounds of the formula I according to one or more of claims 1 to 6, where [A]⁻ can also be selected from [O-S(O)₂-CₘF₂ₘ₊₁]⁻ with m = 1, 2, 3, 4, 5, 6, 7 or 8 and [F_{z}B(CₘF₂ₘ₊₁)_{4-z}]⁻ with z = 4, as additives in electrolytes.

15. The use of compounds of the formula I according to one or more of claims 1 to 6, where [A]⁻ can also be selected from [O-S(O)₂-CₘF₂ₘ₊₁]⁻ with m = 1, 2, 3, 4, 5, 6, 7 or 8 and [F_{z}B(CₘF₂ₘ₊₁)_{4-z}]⁻ with z = 4, in electrochemical or electrooptical devices.

16. The use according to claim 15, wherein the electrochemical or electrooptical device is a lithium battery, a lithium ion battery, a double layer capacitor, a lithium capacitor, a solar cell, an electrochromic display, a sensor or a biosensor.

## Revendications

1. Composés de formule I
[K-(CH₂)-(CH₂)ᵤ-SO₃-(CH₂)ᵥ-R'']⁺[A]⁻
dans laquelle
u signifie 0, 1, 2, 3, 4, 5, 6 ou 7, et au moins un groupe CH₂ de la chaîne alkylène -(CH₂)ᵤ- avec u = 2, 3, 4, 5, 6 ou 7 peut être remplacé par 0 ou peut contenir au moins une double liaison,
v signifie 0, 1, 2, 3 ou 4, -SO₃- signifie 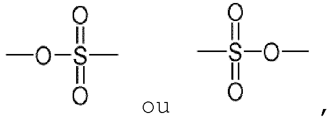 les R'' signifient chacun indépendamment les uns des autres un groupe alkyle linéaire ou ramifié de 1 à 8 atomes C, qui peut être non fluoré, partiellement fluoré ou entièrement fluoré, un groupe alcényle linéaire ou ramifié contenant 2 à 20 atomes C et une ou plusieurs doubles liaisons, un groupe alcynyle linéaire ou ramifié contenant 2 à 20 atomes C et une ou plusieurs triples liaisons, ou un groupe aryle de 6 à 12 atomes C, qui peut être substitué une ou plusieurs fois par F, Cl et/ou un groupe alkyle linéaire ou ramifié, partiellement fluoré ou entièrement fluoré, de 1 à 8 atomes C,
K est un cation choisi dans le groupe constitué par
R₃N⁺-_{*}, R₃P⁺-_{*}, 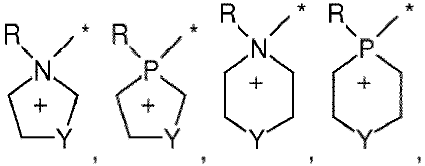 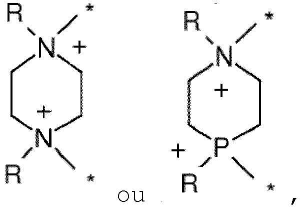
les R signifiant chacun indépendamment les uns des autres méthyle, éthyle, n-propyle, iso-propyle, n-butyle, sec.-butyle ou tert.-butyle, au moins un groupe CH₂ non relié directement avec N ou P des radicaux R mentionnés pouvant être remplacé par 0,
Y signifiant CH₂, 0, S ou NR', et
R' signifiant méthyle, éthyle, n-propyle, iso-propyle, n-butyle, sec.-butyle ou tert.-butyle,
A est un anion choisi dans le groupe constitué par
[F_{z}B(CₘF₂ₘ₊₁)_{4-z}]⁻,
(F_{y}P(CₘF₂ₘ₊₁)_{6-y}]⁻,
[(CₘF₂ₘ₊₁)₂P(O)O]⁻,
[CₘF₂ₘ₊₁P(O)O₂]²⁻,
[O-C(O)-CₘF₂ₘ₊₁]⁻,
[N(C(O)-CₘF₂ₘ₊₁)₂]⁻,
[N(S(O)₂-CₘF₂ₘ₊₁)₂]⁻,
[N(C(O)-CₘF₂ₘ₊₁)(S(O)₂-CₘF₂ₘ₊₁)]⁻,
[N(C(O)-CₘF₂ₘ₊₁)(C(O)F)]⁻,
[N(S(O)₂-CₘF₂ₘ₊₁)(S(O)₂F)]⁻,
[N(S(O)₂F)₂]⁻,
[C(C(O)-CₘF₂ₘ₊₁)₃]⁻,
[C(S(O)₂-CₘF₂ₘ₊₁)₃]⁻, 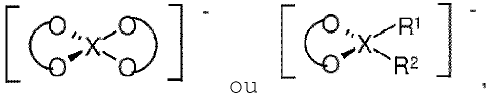
m signifiant 1, 2, 3, 4, 5, 6, 7 ou 8, des groupes CF₂ dans les anions mentionnés pouvant en partie être remplacés par O, S(O)₂, NR ou CH₂,
z signifiant 1, 2 ou 3,
y signifiant 1, 2, 3, 4, 5 ou 6,
X signifiant B ou Al,
R¹ et R² signifiant chacun indépendamment l'un de l'autre F, Cl, Br, I, un groupe perfluoroalkyle linéaire ou ramifié de 1 à 20 atomes C, un groupe alcoxy linéaire ou ramifié de 1 à 20 atomes C, qui peut être non fluoré, partiellement fluoré ou entièrement fluoré, ou -O-C(O)-alkyle, alkyle signifiant un groupe alkyle linéaire ou ramifié de 1 à 20 atomes C, qui peut être non fluoré, partiellement fluoré ou perfluoré,
et les 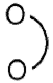 signifiant chacun indépendamment les uns des autres un radical bidentate, qui dérive d'un 1,2- ou 1,3-diol, d'un acide 1,2- ou 1,3-dicarboxylique, ou d'un acide 1,2- ou 1,3-hydroxycarboxylique, dans lequel deux groupes OH voisins du composé en question forment chacun une liaison avec l'atome central X et deux atomes H sont formellement clivés,
et l'électroneutralité du sel de formule I correspondant devant être respectée.

2. Composés selon la revendication 1, **caractérisés en ce que** v signifie 0.

3. Composés selon la revendication 1 ou 2, **caractérisés en ce qu'**u signifie 1, 2 ou 3.

4. Composés selon une ou plusieurs des revendications 1 à 3, **caractérisés en ce que** R" signifie un groupe alkyle linéaire ou ramifié de 1 à 8 atomes C, qui peut être non fluoré, partiellement fluoré ou entièrement fluoré.

5. Composés selon une ou plusieurs des revendications 1 à 4, **caractérisés en ce que** K correspond au cation 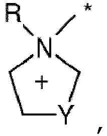 [A]⁻ pouvant également être choisi parmi [O-S(O)₂-CₘF₂ₘ₊₁]⁻ avec m = 1, 2, 3, 4, 5, 6, 7 ou 8, et [_{F}zB(CₘF₂ₘ₊₁)_{4-z}]⁻ avec z = 4.

6. Composés selon une ou plusieurs des revendications 1 à 5, **caractérisés en ce qu'**A est un anion choisi dans le groupe constitué par
[F_{z}B(CₘF₂ₘ₊₁)_{4-z}]⁻,
[F_{y}P(CₘF₂ₘ₊₁)_{6-y}]⁻,
[O-C(O)-CₘF₂ₘ₊₁]⁻,
[N(S(O)₂-CₘF₂ₘ₊₁)₂]⁻,
[N(S(O)₂F)₂]⁻, 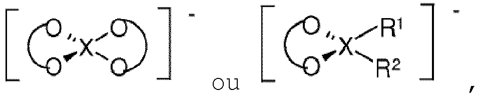
m signifiant 1, 2, 3, 4, 5, 6, 7 ou 8,
z signifiant 1, 2 ou 3,
y signifiant 3, 4, 5 ou 6,
X signifiant B,
R¹ et R² signifiant chacun indépendamment l'un de l'autre F, un groupe perfluoroalkyle linéaire ou ramifié de 1 à 4 atomes C, un groupe alcoxy linéaire ou ramifié de 1 à 4 atomes C, ou -O-C(O)-alkyle, alkyle signifiant un groupe alkyle linéaire ou ramifié de 1 à 20 atomes C, qui peut être non fluoré, partiellement fluoré ou perfluoré,
et les 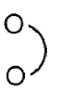 signifiant chacun indépendamment les uns des autres un radical bidentate, qui dérive d'un 1,2- ou 1,3-diol, d'un acide 1,2- ou 1,3-dicarboxylique, ou d'un acide 1,2- ou 1,3-hydroxycarboxylique, dans lequel deux groupes OH voisins du composé en question forment chacun une liaison avec l'atome central X et deux atomes H sont formellement clivés.

7. Procédé de fabrication de composés de formule I selon une ou plusieurs des revendications 1 à 6, dans lesquels SO₃ correspond à la structure partielle 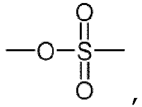 **caractérisé en ce qu'**un composé de formule (II)
K-1 (II)
K-1 étant choisi dans le groupe constitué par R₃N, R₃P, 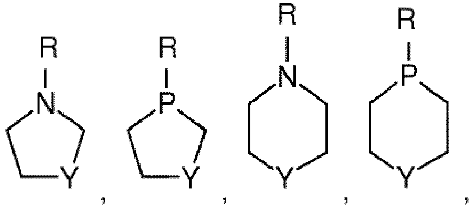 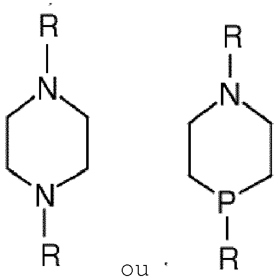
les R signifiant chacun indépendamment les uns des autres méthyle, éthyle, n-propyle, iso-propyle, n-butyle, sec.-butyle ou tert.-butyle, au moins un groupe CH₂ non relié directement avec N ou P des radicaux R mentionnés pouvant être remplacé par O,
Y signifiant CH₂, O, S ou NR', et
R' signifiant méthyle, éthyle, n-propyle, isopropyle, n-butyle, sec.-butyle ou tert.-butyle,
est tout d'abord mis en réaction avec un composé de formule (III)
Hal-(CH₂)-(CH₂)ᵤ-OH (III)
dans laquelle u signifie 1, 2, 3, 4, 5, 6 ou 7, au moins un groupe CH₂ non relié à l'oxygène de la chaîne alkylène -(CH₂)ᵤ- avec u = 2, 3, 4, 5, 6 ou 7 pouvant être remplacé par O, ou pouvant contenir au moins une double liaison, et Hal signifiant Cl, Br ou I,
et le composé intermédiaire de formule (IV) formé
[K-(CH₂)-(CH₂)ᵤ-OH]⁺ [Hal]⁻ (IV)
dans laquelle K, u et Hal ont une des significations indiquées précédemment,
est mis en réaction avec un composé de formule (V)
R''-(CH₂)ᵥ-SO₂-L (V)
dans laquelle v et R'' ont une signification selon la revendication 1, et L signifie un groupe partant choisi parmi un groupe alcoxy linéaire ou ramifié de 1 à 3 atomes C, Cl ou F,
sous catalyse acide dans le cas du groupe alcoxy en tant que groupe partant ou en présence d'une base dans le cas de Cl ou F en tant que groupe partant,
et le composé de formule (VI) formé
[K-(CH₂)-(CH₂)ᵤ₋SO₃(CH₂)ᵥ-R"]⁺ [Hal]⁻ (VI)
dans laquelle K, u, v, R" et Hal ont une des significations indiquées précédemment,
est mis en réaction dans une réaction de métathèse avec un composé de formule (VII)
[Kt]⁺[A]⁻ (VII)
dans laquelle [Kt]⁺ signifie un cation de métal alcalin ou H⁺, et [A]⁻ a une signification indiquée dans la revendication 1.

8. Procédé de fabrication de composés de formule I selon une ou plusieurs des revendications 1 à 6, dans lesquels SO₃ correspond à la structure partielle 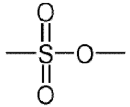 et u signifie 2, 3, 4 ou 5, **caractérisé en ce qu'**un composé de formule (II)
K-1 (II)
K-1 étant choisi dans le groupe constitué par R₃N, R₃P, 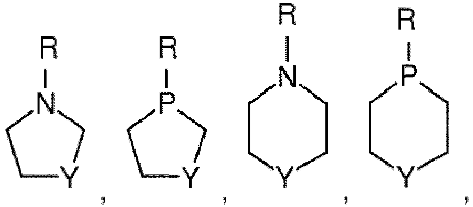 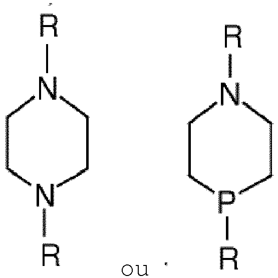
les R signifiant chacun indépendamment les uns des autres méthyle, éthyle, n-propyle, iso-propyle, n-butyle, sec.-butyle ou tert.-butyle, au moins un groupe CH₂ non relié directement avec N ou P des radicaux R mentionnés pouvant être remplacé par 0,
Y signifiant CH₂, O, S ou NR', et
R' signifiant méthyle, éthyle, n-propyle, isopropyle, n-butyle, sec.-butyle ou tert.-butyle,
est tout d'abord mis en réaction avec un composé de formule (VIII) 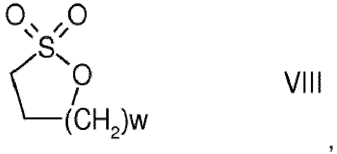 dans laquelle w signifie 1, 2, 3 ou 4,
pour former le composé intermédiaire de formule (IX)
[K-(CH₂)-(CH₂)ᵤ-SO₃⁻] (IX)
dans laquelle K et u ont la signification indiquée dans la revendication 1, puis le composé intermédiaire de formule (IX) est mis en réaction avec un agent d'alkylation de formule (X)
L*-(CH₂)ᵥ-R" (X)
dans laquelle v et R" ont une signification indiquée dans la revendication 1, et L* signifie CF₃-S(O)₂O, C₄F₉-S(O)₂O, (C₂F₅)₂P(O)O, (C₄F₉)₂P"(O)O, (alkyle) ₂O⁺, alkyle-S(O)₂O, alkyle-O-S(O)₂O, I ou Br, les alkyles signifiant chacun indépendamment les uns des autres un groupe alkyle linéaire ou ramifié de 1 à 4 atomes C,
et le composé de formule (XI) obtenu
[K-(CH₂)-(CH₂)ᵤ-SO₃(CH₂)ᵥ-R'']⁺[L*]⁻ (XI)
dans laquelle K, u, v, R'' et L* ont une signification indiquée précédemment,
est éventuellement mis en réaction avec un composé de formule (VII)
[Kt]⁺[A]⁻ (VII)
dans laquelle [Kt]⁺ signifie un cation de métal alcalin ou H⁺, et [A]⁻ a une signification indiquée dans la revendication 1 lorsque l'anion [L*]⁻ ne correspond pas déjà à une signification de l'anion [A]⁻.

9. Procédé de fabrication de composés de formule I selon une ou plusieurs des revendications 1 à 6, dans lesquels SO₃ correspond à la structure partielle 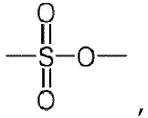 **caractérisé en ce qu'**un composé de formule (II)
K-1 (II)
K-1 étant choisi dans le groupe constitué par R₃N, R₃P, 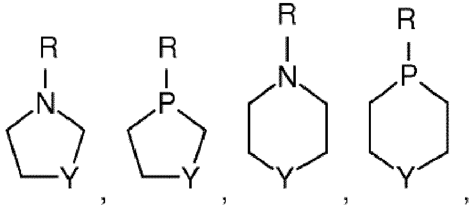 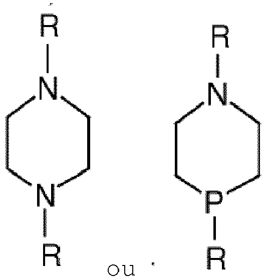
les R signifiant chacun indépendamment les uns des autres méthyle, éthyle, n-propyle, iso-propyle, n-butyle, sec.-butyle ou tert.-butyle, au moins un groupe CH₂ non relié directement avec N ou P des radicaux R mentionnés pouvant être remplacé par 0,
Y signifiant CH₂, O, S ou NR', et
R' signifiant méthyle, éthyle, n-propyle, isopropyle, n-butyle, sec.-butyle ou tert.-butyle,
est tout d'abord mis en réaction avec un composé de formule (XII)
Hal-(CH₂)-(CH₂)ᵤ-Hal (XII)
dans laquelle u signifie 0, 1, 2, 3, 4, 5, 6 ou 7, au moins un groupe CH₂ non relié à Hal de la chaîne alkylène -(CH₂)ᵤ- avec u = 2, 3, 4, 5, 6 ou 7 pouvant être remplacé par O ou pouvant contenir au moins une double liaison, et Hal signifie Cl, Br ou I,
et le composé intermédiaire de formule (XIII) formé
[K-(CH₂)-(CH₂)ᵤ-Hal]⁺[Hal]⁻ (XIII)
dans laquelle K, u et Hal ont une des significations indiquées précédemment,
est mis en réaction avec un sulfite de métal alcalin ou du sulfite d'ammonium, puis avec un composé de formule (X)
L*-(CH₂)v-R" (X)
dans laquelle v et R" ont une signification indiquée dans la revendication 1, et L* signifie CF₃-S(O)₂O, C₄F₉-S(O)₂O, (C₂F₅) ₂P(O)O, (C₄F₉)₂P(O)O, (alkyle) ₂O⁺, alkyle-S(O)₂O, alkyle-O-S(O)₂O, I ou Br, les alkyles signifiant chacun indépendamment les uns des autres un groupe alkyle linéaire ou ramifié de 1 à 4 atomes C,
et le composé de formule (XI) formé
[K-(CH₂)-(CH₂)ᵤ-SO₃(CH₂)ᵥ-R'']⁺[L*]⁻ (XI)
dans laquelle K, u, v, R'' et L* ont une des significations indiquées précédemment,
est éventuellement mis en réaction avec un composé de formule (VII)
[Kt]⁺[A]⁻ (VII)
dans laquelle [Kt]⁺ signifie un cation de métal alcalin ou H⁺, et [A]⁻ a une signification indiquée dans la revendication 1 lorsque l'anion [L*]⁻ ne correspond pas déjà à une signification de l'anion [A]⁻.

10. Électrolyte, contenant au moins un composé de formule I selon une ou plusieurs des revendications 1 à 6, dans lequel [A]⁻ peut également être choisi parmi [O-S(O)₂-CₘF₂ₘ₊₁]⁻ avec m = 1, 2, 3, 4, 5, 6, 7 ou 8, et [F_{z}B(CₘH₂ₘ₊₁)_{4-z}]⁻ avec z = 4.

11. Électrolyte selon la revendication 10, **caractérisé en ce qu'**un sel de lithium et/ou un sel de tétraalkylammonium sont contenus en tant que sel conducteur.

12. Électrolyte selon la revendication 10 ou 11, **caractérisé en ce qu'**un ou plusieurs additifs sont contenus.

13. Dispositif électrochimique ou électrooptique, contenant au moins un composé de formule I selon une ou plusieurs des revendications 1 à 6, dans lequel [A]⁻ peut également être choisi parmi [O-S(O)₂-CₘF₂ₘ₊₁]⁻ avec m = 1, 2, 3, 4, 5, 6, 7 ou 8, et [F_{z}B(CₘF₂ₘ₊₁)_{4-z}]⁻ avec z = 4.

14. Utilisation de composés de formule I selon une ou plusieurs des revendications 1 à 6, dans lesquels [A]⁻ peut également être choisi parmi [O-S(O)₂-CₘF₂ₘ₊₁]⁻ avec m = 1, 2, 3, 4, 5, 6, 7 ou 8, et [F_{z}B(CₘF₂ₘ₊₁)_{4-z}]⁻ avec z = 4, en tant qu'additif dans des électrolytes.

15. Utilisation de composés de formule I selon une ou plusieurs des revendications 1 à 6, dans lesquels [A]⁻ peut également être choisi parmi [O-S(O)₂-CₘF₂ₘ₊₁]⁻ avec m = 1, 2, 3, 4, 5, 6, 7 ou 8, et [F_{z}B(CₘH₂ₘ₊₁)_{4-z}]⁻ avec z = 4, dans des dispositifs électrochimiques ou électrooptiques.

16. Utilisation selon la revendication 15, **caractérisée en ce que** le dispositif électrochimique ou électrooptique est une batterie au lithium, une batterie aux ions lithium, un condensateur double couche, un condensateur au lithium, une cellule solaire, un affichage électrochrome, un capteur ou un biocapteur.
